# EUROPEAN PATENT APPLICATION

(11) **EP 1 618 874 A2**
(43) Date of publication of application: **25.01.2006**
(21) Application number: 04730557.8
(22) Date of filing: 30.04.2004
(51) Int. Cl.: A61K 31/00, A61K 31/366, A61K 31/22, A61K 31/40, A61K 31/404, A61K 45/06, A61P 31/12, A61P 31/18, A61K 38/17

(54) **PREVENTION OF HIV-1 INFECTION BY INHIBITION OF RHO-MEDIATED REORGANISATION AND/OR CONTENT ALTERATION OF CELL MEMBRANE RAFT DOMAINS**

(30) Priority: 30.04.2003 US 466429 P
(71) Applicant: CONSEJO SUPERIOR DE INVESTIGACIONES CIENTIFICAS, 28006 Madrid (ES); Pharmacia Spain S.A., 08190 Sant Cugat de Valles (Barcelona) (ES)
(72) Inventor: MELLADO, Mario, 28008 Madrid (ES); RODRIGUEZ FRADE, José, Miguel, 28016 Madrid (ES); MARTINEZ ALONSO, Carlos, 28034 Madrid (ES); MANES, Santos, 28005 Madrid (ES); DEL REAL, Gustavo, 28240 Madrid (ES)
(74) Representative: Elzaburu Marquez, Alberto
(86) International application number: PCT/ES2004/000188
(87) International publication number: WO 2004/096194

(57) **Abstract**

The present invention relates generally to the prevention or delaying of retroviral infection by use of agents that prevent the clustering of retroviral receptors associated with cell membrane raft domains. The present invention relates more specifically to the prevention or treatment of HIV-1 infection through the use of agents that inhibit Rho-A activation by affecting GTPase activity or protein isoprenylation. The present invention relates also to the prevention or delay of HIV-1 infection through the displacement of cytokine receptors from cell membrane raft domains.

## Description

### PRIORITY

This application claims the priority of U.S. provisionnal application serial 60/466,429, filed April 30, 2003.

### TECHNICAL FIELD

The present invention relates generally to the prevention or delaying of retroviral infection by administration of agents that prevent the clustering of retroviral receptors associated with cell membrane raft domains. The present invention relates more specifically to the prevention or treatment of HIV-1 infection through the use of agents that inhibit Rho-A activation by affecting GTPase activity or protein isoprenylation. The present invention relates also to the prevention or delay of HIV-1 infectiofn through the displacement of cytokine receptors from cell membrane raft domains. More particularly, the present invention relates to the use of protein isoprenylation inhibitors in the treatment of HIV infection, such as HIV-1, and genetically related retroviral infections (and the resulting acquired immune deficiency syndrome, AIDS).

### BACKGROUND

The information provided below is not admitted to be prior art to the present invention, but is provided solely to assist the understanding of the reader.

The plasma membrane is a specialized structure that channels and integrates the information that flows continuously between a cell and its environment. The cell plasma membrane also constitutes the initial barrier against infection by intracellular pathogens. Contrary to the view of the plasma membrane as a homogenous phospholipid backbone loaded with proteins, the last decade has highlighted the heterogeneity of phases conforming plasma membrane. In particular, accumulated evidence indicates that specialized lipid domains, termed rafts, have fundamental roles in regulating an array of cellular processes, ranging from signal transduction to the gateways for infection with intracellular pathogens (Simons. K. and Toomre, D. (2000) Nat. Rev. Mol. Cell. Biol. 1, 31.-39; Brown, D. and London. E. (2000) J. Biol. Chem. 275, 17221-17224; Manes, S. et al (2001) Semin. Immunol. 13, 147-157; Mellado. M. et al (2001) Annu. Rev. Immunol. 19, 397-421). Current evidence supports a major role of raft domains structure in the regulation of the various interactions between membrane components. Moreover, evidence supports a role for raft domains in the infectivity and propagation of pathogens, particularly including the human immunodeficiency virus (HIV) (Mañes, S. et al. (2003) Nat. Rev. Immunol. 3, 557-568).

The organization of lipids in a membrane is to a large extent determined by the phase of the bilayer. The various phases of lipid bilayers represent physical states, which differ in the packing, the degree of order and the mobility of the constituting lipids (Brown, D. and London, E. (1998) J. Membr. Biol. 164, 101-114; Rietveld, A. and Simons, K. (1998) Biochim. Biophys, Acta 1376, 467-479; Brown, D. (2001) Proc. Nat. Acad. Sci. USA 98, 10517-10518). The two extreme phases are the quasi-solid gel liquid-crystalline (l_{c}) and the liquid-disordered (l_{d}) phase. Homogenous bilayers formed from purified phospholipids or sphingolipids, exhibit a sharp, temperature-dependent transition between gel and disordered phases occurring at a characteristic melting temperature (Tₘ). This phase separation in the membrane is the consequence of the differential packing ability of sphingolipids and phospholipids. Sphingolipids contain long, largely saturated acyl chains, which confer a much higher Tₘ than is possessed by glycerophospholipids, which are rich in bent, unsaturated acyl chains. Therefore glycerolipid bilayers are in a highly fluid l_{d} phase and their lipids have a high rotational and lateral mobility. In contrast, sphingolipid-enriched membranes are highly ordered, with their lipids densely packed and with strongly reduced mobility in the plane of the bilayer.

In the presence of cholesterol, lipid bilayers can also adopt a third, intermediate phase termed liquid-ordered phase (*l*_{*o*}). When present in a bilayer, cholesterol tends to occupy the spaces between the saturated hydrocarbon chains of the lipids (Smaby, J. et al (1996) Biochemistry 35, 5696-5704). By aligning with the phospholipids and sphingolipids, the presence of cholesterol in membranes increases the order of hydrocarbon chains but, importantly, reduces the formation of gel phases (Bittman, R. (1997) Subcell. Biochem. 28, 145-171). Consequently, the *l*ₒ phase is characterized by a substantial lateral and rotational lipid mobility. Model membrane studies support the idea that *l*_{*o*} and *l*_{*c*} phases could co-exist in biological membranes. In these artificial bilayers, cholesterol partitions preferentially with high *T*ₘ lipids into *l*ₒ membrane domains whereas it segregates from *l*_{c} phase domains enriched in low *T*ₘ lipids (Sanharam, M. and Thompson, T. (1991) Proc. Natl. Acad. Sci. USA 88, 8686-8690; Ahmed, S. et al (1997) Biochemistry 36, 10944-10953. Accordingly, in complex lipid bilayers, such as the plasma membrane, cholesterol is believed to assemble with lipids containing saturated hydrocarbon chains into raft membrane domains (Ge, M. et al (1999) Biophys. J. 77, 925-933). Raft domains may be therefore defined as membranes in the *l*ₒ phase -or a state with similar properties- resulting from the preferential lateral packing of high *T*ₘ lipids and cholesterol in the external leaflet of the bilayer.

The raft hypothesis states that separation of discrete liquid-ordered and liquid-disordered phase domains occurs in membranes containing sufficient amounts of sphingolipid and sterol such as cell plasma membrane. Although studies in artificial membranes support this concept, the existence of rafts has not yet been however conclusively demonstrated in cell membranes. Nevertheless, several approaches strongly suggest that cell membranes do contain rafts. These methods include cholesterol-dependent detergent insolubility and co-localization of independently clustered proteins and lipids in patches on the cell surface (Brown, D. and London. E. supra), fluorescence resonance energy transfer (FRET) between Glycosylphosphatidyl inositol (GPI)-anchored proteins (Varma, R. and Mayor, S. (1998) Nature 394, 798-801; Kenworthy, A. et al (2000) Mot. Biol. Cell 11. 1645-1655), single-particle tracking (Pralle, A. et al (2000) J. Cell Biol.48, 997-1007), and single-molecule microscopy (Schütz, G., Kada, G., Pastushenko, V. and Schindler, H. (2000) EMBO J. 19, 892-901). Estimates of raft size vary from a few nanometers to microns (Varma, R. and Mayor, S. supra; Kenworthy, A. et al supra; Pralle, A. et al supra; Schütz, G. et al supra). This suggests that rafts in cells are likely more complex than those in model membranes.

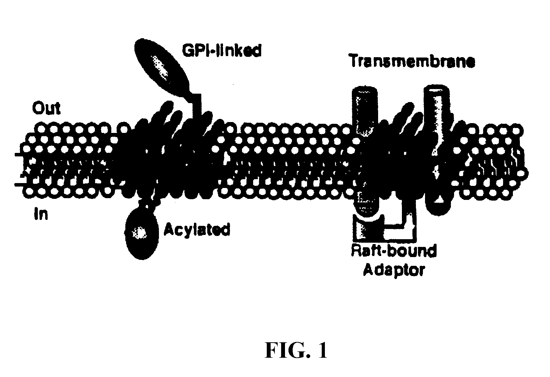

A frequently used biochemical approach to identify raft domains in cellular membranes is the purification of detergent-resistant membranes (DRM) in flotation density gradients. This method is based in the relative insolubility of lipid bilayers in a *l*ₒ phase in non-ionic detergents such as Triton X-I 00 at low temperatures (Brown, D. and London, E. (1997) Biochem. Biophys. Res. Commun. 240, 1-7). This property has been demonstrated in *l*_{*o*}/*l*_{*d*} biphasic artificial membrane systems, in which detergent insolubility correlates with the content of the *l*_{*o*} membrane phase (Dietrich, C. et al (2001) Biophys. J. 80, 1417-1428). Studies in artificial membranes also indicate that some of the abundant DRM lipids such as cholesterol are actually required to maintain and/or assemble *lo* domains (Dietrich, C. et al supra). In addition to their specific lipid composition, DRMs are enriched in several classes of proteins (Simons. K. and Toomre, D. supra) (Fig. 1). GPI-anchored proteins constitute one of major class of raft-associated proteins linked to the exoplasmic plasma membrane leaflet via a phosphatidyl inositol moiety. The GPI's acyl and alkyl chains are generally saturated in accordance with their high affinity for *l*ₒ membranes and raft associations (Dietrich, C. et al (2001) Proc. Natl. Acad. Sci. USA 98. 10642-10647). Doubly acylated cytoplasmic proteins are another major class of DRM-associated proteins. These include specific members of the src-kinases family and certain types of G_{α} subunits of heterotrimeric GTPases. These proteins are thought to anchor via their acyl chains, most likely saturated palmitic acid (Resh, M. (1999) Biochim. Biophys. Acta 1451, 1-16; Liang, X. et al (2001) J. Biol. Chem. 276, 30987-30994). In contrast, both membrane-spanning and prenylated proteins are difficult to accommodate in an ordered environment. Indeed, DRM are relatively poor in transmembrane proteins and contain very low levels of prenylated proteins (Melkonian, K. et al (1999) J. Biol. Chem. 274, 3910-3917). Some transmembrane proteins are also enriched in DRM, however. Palmitoylation contributes to DRM targeting for some transmembrane proteins (Melkonian, K. et al (1999) supra), although not all palmitoylated transmembrane proteins are in DRM. Moreover, the sequence of the membrane-spanning domain can affect DRM partitioning (Perschl, A. et la (1995) J. Cell Sci. 108, 1033-1041; Scheiffele, P. et al (1997) EMBO J. 16, 5501-5508). However, mutations in cytoplasmic domains can also affect DRM association of transmembrane proteins (Polyak, M. et al (1998) J. Imrnunol. 161, 3242-3248), even though the cytoplasmic tails probably do not interact directly with the lipid bilayer. A possibility to explain these observations is that such cytoplasmic mutants fail to interact with binding partners, which target and/or anchor the native transmembrane proteins to lipid rafts (Brückner, K. et al (1999) Neuron 22, 511-524; Oliferenko. S. et al (1999) J. Cell Biol. 146, 843-854; Machleidt, T. et al (2000) J. Cell Biol. 148, 17-28).

DRM represent post-lysis membrane aggregations and therefore it is difficult to make quantitative comparisons between DRMs and native rafts (London, E. and Brown, D. (2000) Biochim. Biophys. Acta 1508, 182-195). As commented above, different new technical approaches have been used to analyze the structure and the dynamics of rafts in living and/or fixed cells. Single particle tracking using colloidal gold conjugated antibodies demonstrated that the ganglioside GM 1 and the raft-associated protein, Thy-1, were transiently confined to zones within the plasma membrane (Sheets, E. et al (1997) Biochemistry 36, 12449-12458). Recently, the measurement of the lateral motion of single molecules, either lipids (Schütz, G. et al (2000) EMBO J. 19, 892-901) or proteins (Pralle, A. et al (2000) J. Cell Biol.48, 997-1007) associated or not to rafts, demonstrated the confinement of specific markers in liquid-ordered membranes at the surface of living cells. The partitioning of the molecules in and out of rafts in these studies was highly dynamic, but at least some raft proteins resided within rafts for up to several minutes.

Whereas the confinement zones detected by single molecule tracking is consistent in independent experiments, FRET analysis of raft markers provide however controversial results. Indeed, proximity measurements between GPI-anchored proteins in some instances indicated that raft-associated markers are concentrated in microdomains of the plasma membrane in a cholesterol sensitive manner (Varma, R. and Mayor, S. (1998) Nature 394, 798-801; De Angelis, D. et al (1998) Proc. Natl. Acad. Sci. USA 95. 12312-12316). In other studies, however, FRET detected between GPI-anchored proteins and the glycosphingolipid GM1 correlated with surface density of the raft marker (Kenworthy, A. et al (2000) Mol. Biol. Cell 11. 1645-1655), a fmding that is inconsistent with clustering in microdomains. Therefore, although some raft markers could be in sub micrometer proximity in the membrane, they are not confined in stabilized rafts. The discrepancies have not been resolved yet.

An approach used widely to probe raft presence in living cells is the manipulation of the levels of lipids forming the rafts. In some cases this strategy is used as a "functional approach" to demonstrate raft involvement in a particular cellular function. Cholesterol depletion is a well-documented method to disrupt raft domain structure (Simons. K. and Toomre, D. (2000) supra). Sterol-binding drugs, toxins or detergents can sequester cholesterol. Cyclodextrins are often used to acutely lower cholesterol levels or, when complexed to cholesterol, to introduce excess cholesterol into cell membranes (Keller, P. and Simons, K. (1998) J. Cell Biol. 140, 1357-1367). Nonetheless, cyclodextrin treatment must be performed for short times since the drug affects both the plasma membrane and the intracellular organelles connected to them when applied for several hours to cells (Hansen, G. et al. (2000) J. Biol. Chem. 275, 5136-5142; Grimmer, S. et al (2000) Mol. Biol. Cell 11, 4205-4216).

Lowering of sphingolipid levels at the plasma membrane by cell treatment with sphingolipid biosynthesis inhibitors or by degradation with bacterial sphingomyelinase, also disrupts DRM association of several raft markers (Scheiffele, P. et al (1997) supra; Hanada, K. et al (1995) J. Biol. Chem. 270, 6254-6260). Notably, DRMs can still form in the absence of glycosphingolipids. This was shown using a cell line deficient in glycosphingolipid synthesis (Ostermeyer. A. et al (1999) J. Biol. Chem. 274, 34459-34466). Interestingly, these cells produced increased amounts of sphingomyelin, possibly to compensate for the reduction of raft-preferring lipids and to maintain ordered raft membrane domains. Overall, alterations in the sphingolipid status of the cell are not as predictable as for cholesterol.

The evidence discussed above pictures the plasma membrane as a dynamic equilibrium between domains in the ordered raft and disordered non-raft phases. However, the use of detergents others than Triton X-100 has suggested that mammalian cells may have different raft subtypes on the surface. This intriguing possibility comes from the seminal observation by Madore and coworkers (Madore, N. et al (1999) EMBO J. 18.6917-6926) showed that two glycosylphosphatidyl inositol-anchored proteins of neuronal cells, Thy-1 and prion protein, could be assigned to membranes differentially extracted by Triton X-100 and Brij 96. Likewise, in epithelial cells it has been described the presence of two raft subtypes based on differential detergent insolubility; the Lubrol-insoluble (but Triton X-100-soluble), and the Triton X-100-insoluble rafts (Roper, K. et al (2000) Nat. Cell Biol. 2. 582-592). Although the integrity of both of the proposed raft subtypes is dependent on cholesterol, the potential dependence of these domains on various sphingolipid species is presently unknown.

In T lymphocytes, however, two different raft subtypes have been identified based on glycosphingolipid composition. Studying chemoattractant-induced polarization of T cells, Gomez-Mouton and coworkers (Gómez-Mouton, C. et al (2001) Proc. Natl. Acad. Sci. USA 98, 9642-9647) found that membrane proteins, such as the chemokine receptor CXCR4 or the plasminogen receptor, partition specifically in GM3-enriched rafts whereas other proteins, such as intercellular adhesion molecules ICAM-I or CD44, partition mostly in GMI-enriched rafts. Both GMI- and GM3-enriched rafts are equally sensitive to cholesterol extraction but also equally resistant to extraction with different detergents. It is important to indicate that cells use the segregation of proteins to different raft subtypes to target specific proteins to spatially restricted membrane locations. Indeed, a high density of prion-protein-enriched rafts is found at the cell body, whereas Thy-1-containing rafts are found mostly in neurites (Madore, N. et al (1999) EMBO J. 18.6917-6926); the lubrol-insoluble rafts in epithelial cells are selectively associated to microvilli, but remains segregated from the planar, Triton-insoluble ones (Roper, K. et al (2000) Nat. Cell Biol. 2. 582-592); finally, GM3-enriched rafts transport specific membrane and cytoskeletal proteins to the leading edge of migrating lymphocytes, whereas GMI-based rafts carry cell-cell adhesion receptors to the uropod, at rear of T cells.

Although rafts were first proposed as a sorting signal in the trans-Golgi network to organize proteins and lipids in specific cell surfaces of polarized epithelial and neuronal cells (Simons. K. and Toomre, D. (2000) supra; Rodriguez-Boulan, E. and Gonzalez, A. (1999) Trends Cell Biol. 9, 291-294), it has become increasingly apparent that lipid rafts exert a multifaceted influence on different cell processes including proliferation (Inokuchi, J. et al (2000) Glycoconj. J. 17, 239-245), apoptosis (Grassme. H. et al (2001) J. Biol. Chem. 276, 20589-20596), migration (Manes, S. et al (1999) EMBO J. 18, 6211-6220; Khanna, K. et al (2002) J. Clin. Invest. 109, 205-211), adhesion (Krauss, K. and Altevogt, P. (1999) J. Biol. Chem, 274, 36921-36927; Lacalle, R. et al (2002) J. Cell Biol. 157, 277-289), and infection by pathogens (van der Goot, F. and Harder, T. (2001) Semin. Immunol. 13, 89-97), among others (for a more general review see Simons. K. and Toomre, D. (2000) supra; Brown, D. and London. E. (2000) J. Biol. Chem. 275, 17221-17224; Mañes, S. et al. (2003) Nat. Rev. Immunol. supra). Raft domains participate in these physiological actions mostly by the spatial and temporal regulation of the protein-protein interactions required to accomplish these processes. Indeed, an important general characteristic of rafts seems to be the stabilization of multiple weak interactions upon a stimulus (Simons. K. and Toomre, D. (2000) supra). This property, together with the high mobility of raft units in the plane of the membrane, may facilitate the interaction (or increase the efficiency) between different signal transduction partners or, as reviewed below, between different cellular receptors involved in pathogen or toxin entry.

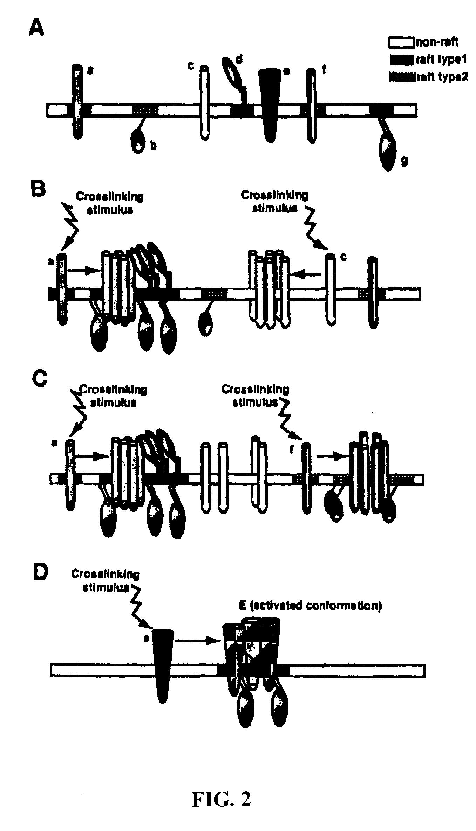

Several possibilities can be envisioned by which rafts function as devices controlling membrane protein-protein interactions (Fig. 2). Considering the small size of rafts, a given raft can only contain a low number of proteins. Therefore, an important step in the initiation of these processes is capacity of individual small rafts to cluster in larger rafts. This coalescence process brings raft-associated components of the machinery together into a larger platform, where may occur the encounter between two previously separated raft proteins. These larger rafts may be more stable than smaller raft domains; raft proteins may induce stabilization of the underlying actin cytoskeleton (Oliferenko. S. et al (1999) J. Cell Biol. 146, 843-854; Villalba, M. et al (2001) J. Cell Biol. 155, 331-338), which could further amplify the attractive forces and promote the assembly of functional complexes eliciting a coordinated response (Lacalle, R. et al (2002) supra). Importantly, the clusters of raft-associated proteins remain segregated from clusters of non-raft membrane proteins when both protein types are artificially-induced to co-patch (Harder. T. et al (1998) J. Cell Biol. 141, 929-942). Therefore, the spatial segregation of proteins into membranes with different phase separation can prevent interactions between raft and non-raft components. Moreover, raft-associated proteins may be separated in different raft subtypes that contain specific set of proteins. As discussed above, raft coalescence seems to occur only between the same raft subtype. Therefore, protein-protein interactions at the plasma membrane may be controlled not only by the spatial segregation of components between raft and non-raft but also by the partitioning of each component into specific raft subtypes.

Rafts may also control interactions between proteins by sequestering a particular membrane element, residing initially in a less ordered region of the membrane, into a pre- existing raft domain. This could occur by dragging a protein into rafts by protein- mediated interactions or by changing its affinity for liquid-ordered phases. Oligomerization is a well-documented factor that increases the affinity of a membrane component for rafts (Harder. T. et al (1998) J. Cell Biol. 141, 929-942). Monitoring of rafts in living cells suggests that at least some raft components are in a dynamic equilibrium with non-raft membranes. Consequently, for every raft element there will be a partition coefficient determining the fraction of raft marker that resides inside and outside of rafts. Exchange of raft components with the surrounding non-raft regions would be probably restricted to the boundaries between the membrane domains. As the ratio of surface area to circumference depends on the raft size, the kinetics of the exchange will also depend on the raft size. Therefore, oligomerization of a membrane protein with a high exchange between raft and non-raft membrane regions may stabilize the association of those proteins into raft-ordered membrane domains. Alternatively, rafts may form *de novo* around oligomerized transmembrane proteins, once they are stable enough to reside in these ordered domains.

The entry of enveloped viruses can be divided into three steps: the attachment of the virus to specific cell surface receptor(s), the conformational change in the viral fusion protein, and the viral-host cell membrane fusion reaction itself. The HIV -1 envelope (Env) gp160 protein is a type I integral membrane protein that mediates viral attachment and membrane fusion. Synthesized as a single polypeptide precursor that forms trimers, Env is cleaved to generate two non-covalently associated subunits, gp120 and gp41. The gp120 subunit binds to the primary cell surface receptor for HIV-1, CD4 (Maddon, P. et al (1986) Cell 47, 333-348). Although CD4 binding is a prerequisite for HIV -1 entry, attachment of virus *per se* is insufficient to mediate viral infection. Several members of the chemokine-receptor family have been shown to act as necessary co-receptors for HIV -1 entry (Berger, E. et al (1999) Annu. Rev. Immunol. 17, 657-700). The initial interaction with CD4 promotes conformational changes in gp120 that renders cryptic regions of the viral glycoprotein for additional interaction with a chemokine receptor family member. This second binding event leads to the host and viral membrane fusion by a gp41-mediated process (Berger, E. et al (1999) Annu. Rev. Immunol. 17, 657-700; Doms, R. and Trono, D. (2000) Genes Dev. 14, 2677-2688). Chemokine-receptor usage varies depending on the viral strain and is the primary determinant of viral tropism. Most primary HIV -1 strains use the chemokine receptor CCR5 in conjunction with CD4 for virus entry (termed R5 virus strains). In some individuals, viruses evolve to use a related receptor, CXCR4, either in place of (X4 virus strains) or in addition to CCR5 (R5X4 strains).

Although the discovery of chemokine receptors as essential receptors for HIV entry has provided great explicatory power for understanding viral tropism and pathogenesis, some crucial pieces of the puzzle are still missing. Indeed, some cell-surface molecules modulate susceptibility to HIV -1 infection, even though they do not interact directly with the viral Env. For instance, cross-linking of CD26, CD28 or CD44 increases cell permissiveness to HIV -1, whereas CD38 decreases susceptibility to the infection (Callebaut, C et al (1993) Science 275, 2045-2050; Dukes, C. et al (1995) J. Virol. 69, 4000-4005; Savarino, A. et al (1999) FASEB J. 13. 2265-2276). It is possible that these molecules modulate HIV -1 entry indirectly by regulating viral receptor density. Moreover, the fusion between the viral and the host cell membranes is a cooperative process that requires the sum of multiple CD4-gp120-coreceptor complexes. It is currently estimated that four to six CCR5 receptors (Kuhmann, S. et al (2000) J, Virol. 74, 71305-7015), multiple CD4 molecules (Layne, S. et al (1990) Nature 346, 277-279), and three to six Env trimers are needed to form a fusion pore. It seems, therefore, that HIV -1 infection depends on multiple intermolecular interactions on the cell surface; CD4 bound to gp120 must find the appropriate coreceptor on the cell surface and, thereafter, different CD4-gp 120-coreceptor complexes must cluster to accomplish viral-cell membrane fusion. It logically follows that host cell surface molecules or signaling pathways that promote or prevent these clustering events would impact the rate and efficiency of virus entry. In this sense, actin remodeling mediated by Rho GTPases may play a pivotal role in these clustering events. Indeed, binding of HIV-1 to the cell surface triggers the specific activation of Rho-A, an event crucial for viral entry.

Recent reports suggest that raft domains may serve as a framework in which occur these lateral associations. First, physicochemical studies have shown the direct interaction of the gp120 with defined glycosphingolipids (Harouse, J. et al (1991) Science 253, 320-323; Yahi, N. et al (1992) J. Virol. 66, 4848-4854; Hammache, D. et al (1998) J. Biol. Chem. 273, 7967-7971; Hammache, D. et al (1999) J. Virol. 73. 5244-5248), suggesting that some steps of virus entry occur in raft domains. Moreover, glycosphingolipid synthesis inhibition (Hug, IP. et al (2000) J. Virol. 74, 6377-6385) and anti-glycosphingolipid antibodies (Harouse, J. et al (1991) Science 253, 320-323) prevent HIV -1 infection *in vitro,* indicating that gp 120 interaction with glycosphingolipids is important for virus infection. Second, cell treatment with cholesterol sequestering drugs that disrupt *l*_{*o*} membranes inhibits HIV -1 infection *in vitro* (Manes, S. et al (2000) EMBO Rep. 1, 190-196; Liao, Z. et al (2001) AIDS Res. Hum. Retroviruses 17, 1009-1019) and *in vivo* (Khanna, K. et al (2002) J. Clin. Invest. 109, 205-211), indicating that the dynamics of rafts may dramatically influence the efficiency of virus entry. Third, targeting of CD4 to non-raft membrane fraction impedes HIV -1 entry, although this CD4 mutant binds to viral Env with the same affinity as the wild-type CD4 (del Real, G. et al (2002) J. Exp. Med. supra).

This evidence strongly indicates that viral entry occurs because the HIV receptors partition into the same membrane phase. In this sense, whereas raft association of CD4 is well established (Xavier, R. et al (1998) Immunity 8, 723-732), chemokine receptor raft partitioning is matter of debate. It has been reported that HIV -1 coreceptors CCR5 and CXCR4 co-purify in the DRM fraction after ligand- or gp120- induced clustering (Manes, S. et al (2001) Semin. Immunol. 13, 147-157; Gómez-Mouton, C. et al (2001) Proc. Natl. Acad. Sci. USA 98, 9642-9647; Manes, S. et al (1999) EMBO J. 18, 6211-6220; Manes, S. et al (2000) EMBO Rep. 1, 190-196; Sorice, M. et al (2001) FEBS Letters 506, 55-60). Moreover, CCR5 and CXCR4 signal in rafts (Mellado. M. et al (2001) supra) and are constitutively associated with other raft proteins such as CD4 (Xiao. X. et al (1999) Proc. Natl. Acad. Sci. USA 96, 7496-7501). Notably, co-localization of the CXCR4 with CD4 occurs in the GM3-enriched raft subtype in T cells (Gómez-Mouton, C. et al (2001) Proc. Natl. Acad. Sci. USA 98, 9642-9647; Sorice, M. et al (2001) FEBS Letters 506, 55-60). Finally, inhibition of glycosphingolipid synthesis reduces the CCR5 levels in the cell membrane (Hug, IP. et al (2000) J. Virol. 74, 6377-6385), suggesting that the early association of CCR5 with rafts is necessary for proper receptor transport.

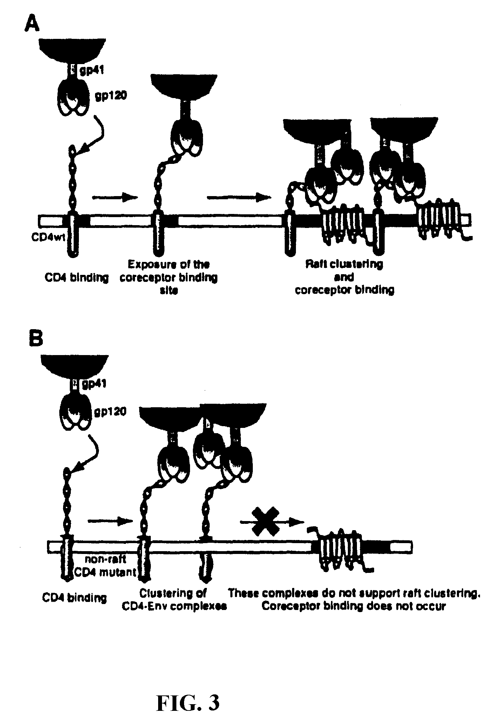

Although several functional evidences suggest that HIV -1 exploits the host raft membrane domain as entry portals, the exact mechanism underlying this process is only being elucidated. Host raft domains may be used by the virus to regulate spatially and temporally the interaction between gp120, CD4 and the co-receptor CXCR4 or CCR5 (Manes, S. et al (2000) EMBO Rep. 1, 190-196; del Real, G. et al (2002) supra) (Fig. 3). According to this model, virus binding to CD4 in rafts induces the lateral diffusion and coalescence of the gp 120/CD4 complexes with rafts containing the co-receptors CXCR4 or CCR5, an actin cytoskeleton-driven process. The strongest evidence supporting this model is the inability to obtain CD4-gp120-coreceptor either in cells expressing the non-raft CD4 mutant or in cholesterol-depleted cells, in which lateral diffusion of rafts is severely impaired. Additionally, some lipids enriched in rafts may be necessary to trigger the supramolecular associations and massive conformational changes in the viral Env required for the formation of the fusion complex (Hug, IP. et al (2000) J. Virol. 74, 6377-6385). Such a model may explain the HIV -co-stimulatory function of CD44 and CD28, since activation of these raft-associated molecules mediates the reorganization of lipid rafts in living cells (Oliferenko. S. et al (1999) J. Cell Biol. 146, 843-854; Viola, A. et al (1999) Science 283, 680-682). Importantly, other viruses such as Ebola virus, also abuse host rafts as entry portals (Bavari, S. et al (2002) J. Exp. Med. 195, 593-602; reviewed in van der Goot, F. and Harder, T. (2001) Semin. Immunol. 13, 89-97). It is tempting therefore to speculate that clustering of raft domains may be a rather general mechanism for cell entry used by distinct intracellular pathogens.

Over a number of years, virologists have observed that the lipid composition of envelope membranes from a variety of viruses, including several retroviruses, is distinct from that of the host plasma membrane from which they are derived, suggesting that they are assembled in membrane micro domains (Aloia, R. et a; (1992), Vol. 6, Wiley-Liss, New York). The HIV envelope, like every biological membrane, is made up of proteins embedded into a lipid bilayer. This bilayer has however a surprisingly high cholesterol/phospholipid molar ratio (>1.00). Sphyngolipids are also enriched in the viral membrane, thus resembling the composition of raft membrane domains (Aloia, R. et al (1993) Proc. Natl. Acad. Sci. USA 90, 5181-5185). Since genes for lipid-metabolizing enzymes are not present in the retrovirus genome, the HIV envelope lipid bilayer is necessarily derived from the host cell lipid membrane.

Recent observations have provided further evidence that new HIV virions emerge from the host cell while wrapping around the raft domains (Nguyen, D. and Hildreth, J. (2000) J. Virol. 74, 3264-3272). Budding of new virions from lipid rafts leads to the exclusion of the cell-membrane abundant CD45 phosphatase from the HIV-1 envelope while other raft-associated molecules, including GPI-anchored proteins Thy-1 and CD59, the Intercellular Adhesion Molecules (ICAM)-1, -2 and -3 (Gómez-Mouton, C. et al (2001) Proc. Natl. Acad. Sci. USA 98, 9642-9647), the integrins LFA-1 and VL-4 (Brown, D. and London. E. (2000) J. Biol. Chem. supra), and the ganglioside GM1 are enriched in the viral particle. The incorporation of host proteins into the viral particle may have a number of consequences on virus infection and pathogenicity, by triggering the activation of signaling pathways in the new infected cells upon viral-cell membrane fusion (Fivaz, M. et al (1999) Trends Cell Biol. 9, 212-213; Liao, Z. et al (2000) AIDS Res. Hum. Retrovir. 16, 355-366). Incorporation of other host membrane glycoproteins, such as HLA-DR class II, which accounts for the 4.4% of the total protein in the viral particle, is probably used by the virus to produce the deregulation of cellular and humoral immune response against HIV-1 (Henderson, L. et al (1987) J. Virol. 61, 629-632).

In addition to the incorporation of raft-associated host proteins into the viral envelope, it has been described the partitioning of structural HIV -1 proteins into the host raft membranes during the assembly of new viral particles. In particular, HIV -1 Gag and Env proteins have been detected in raft domains (Nguyen, D. and Hildreth, J. (2000) J. Virol. 74, 3264-3272; Rousso, I. et al (2000) Proc. Nad. Acad. Sci. USA 97, 13523-13525). The Gag protein of HIV -1 is synthesized as a precursor polyprotein, Pr55Gag, which is composed of matrix (MA), capsid (CA), nucleocapsid (NC), and p6 domains, as well as the spacer peptides p2 and pI (Frankel, A. and Young, J. (1998) Annu. Rev. Biochem. 67, 1-25). It has been reported that raft association of Pr55Gag is initiated by the MA domain attachment to the plasma membrane. This binding step involves, directly or indirectly, at least several domains throughout the MA protein. The specificity of plasma membrane binding is conferred by the M domain, which consist of a combination of an N-terminal myristoyl moiety and a cluster of positively charged residues, which act synergistically (Frankel, A. and Young, J. (1998) Annu. Rev. Biochem. 67, 1-25). Mutation of the N-terminal Gly residue, which serves as the site for myristic acid modification, impaired binding of Gag to membrane and blocked virus assembly (Freed. E. et al (1994) J. Virol. 68, 5311-5320).

Even though acylation is a frequent modification in raft-associated proteins, MA myristoylation is not a major factor to target Pr55Gag to rafts. Studies with Pr55Gag mutants suggest that profuse Gag association with rafts occurs after membrane-bound Gag-Gag interactions. The p2 spacer of Pr55Gag is required for Gag oligomerization (Accola, M. et al (2000) J, Virol. 74, 5395-5402) and, accordingly, Pr55Gag deletion mutants lacking p2 showed an impaired raft association (Ono, A. and Freed, E. (2001) Proc. Natl. Acad. Sci. USA 98, 13925-13930). It is worth noting that a portion of the total Gag protein in infected cells is present in dense raft-like domains, termed barges (Lindwasser, O. and Rosh. M. (2001) J. Virol. 75, 7913-7924). Barges are probably the result of extensive Gag clustering mediated by the NC and p6 domains. It has been proposed that Gag oligomers may display a stronger affmity for rafts because of an increased number of binding sites per complex or an altered conformation induced by Gag-Gag interaction. Gag oligomers may, on the other hand, stabilize raft clusters giving rise to the large and dense barge structures observed.

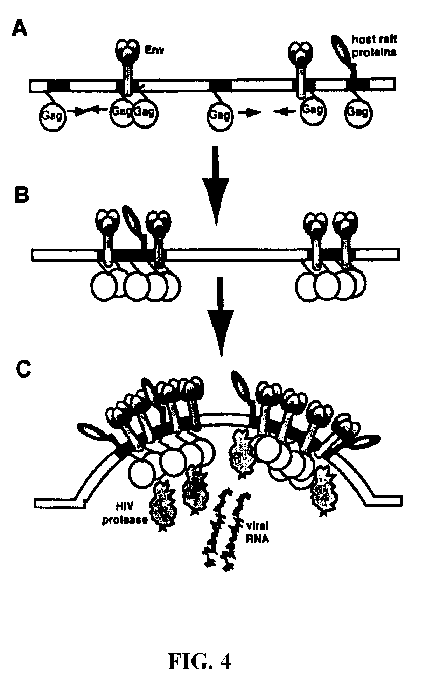

The clustering of Pr55Gag induces the formation of a bud in the membrane and the subsequent release of viral particles (Fig. 4). During budding, the viral Env glycoproteins are incorporated into the new viral particles, a function that is also performed by the MA domain of Pr55Gag. The Env cytoplasmic tail contains two residues, putative targets for modification by palmitoylation. The removal of these amino acids seems not affect either envelope expression, protein trafficking, or Env-Gag interaction (Yang, C. et al (1995) Proc. Natl. Acad. Sci. USA 92. 9871-9875). However, other studies have shown acylation of Env has been shown to be critical for efficient membrane targeting and formation of infectious virus. Therefore, the cytoplasmic domain of gp160 targets the envelope protein to the lipid rafts even in the absence of Gag (Rousso, I. Et al (2000) Proc. Nad. Acad. Sci. USA 97, 13523-13525). It is noted that rafts are concentrated at cell-cell contact sites T cells; therefore, targeting of Env protein to host rafts may facilitate cell-to-cell transmission of HIV -1.

The evidence suggests that rafts may be involved in HIV -1 assembly and release. The functional significance of rafts in the budding of immature HIV -1 virions is highlighted as cholesterol depletion decreases HIV -1 particle production in infected cells (Ono, A. and Freed, E. (2001) Proc. Natl. Acad. Sci. USA 98, 13925-13930; Maziere, J. et al (1994) Biomed. Pharmacother. 48, 63-67). Rafts may function in this process as platforms for Gag oligomerization at the plasma membrane (Ono, A. and Freed, E. (2001) Proc. Natl. Acad. Sci. USA 98, 13925-13930; Lindwasser, O. and Rosh. M. (2001) J. Virol. 75, 7913-7924), which in turn would recruit the viral Env necessary to form new infective particles. Additionally, viral budding may be dependent on specific host raft components, either lipids or proteins, which regulate virion release (Vogt, V. (2000) Proc. Natl. Acad. Sci. USA 97, 12945-12947). This may explain the fact that cholesterol depletion markedly reduces HIV-1 particle production, while this treatment does not affect Gag or Env protein synthesis, processing and/or cell-surface expression.

In addition to structural HIV-1 proteins, the regulatory protein Nef also associates with raft domains (Wang, J.-K. et al (2000) Proc. Natl. Acad. Sci. USA 97, 394-399). This important virulence factor in viral pathogenesis has been reported to use host rafts to prime T cells for activation through CD3 and CD28 receptors (Wang, J.-K. et al (2000) Proc. Natl. Acad. Sci. USA 97, 394-399). In the course of infection, cells are activated and polarized and rafts, which are normally dispersed, tend to coalesce along with GPI -linked proteins and associated intracellular signaling proteins. The raft coalescence induced by Nef thus concentrate mediators of T cell activation, including Lck, Fyn and LAT (Lanzavecchia. A. et al (1999) Cell 96, 1-4) resulting in the initiation of the signaling cascades that promotes IL-2 secretion and stimulation of HIV-1 transcriptional activity (Doms, R. and Trono, D. (2000) Genes Dev. 14, 2677-2688).

In summary, HIV uses raft domains to concentrate viral Env and Gag proteins, which constitute only a small fraction of the total proteins in the host cell, into relatively small areas; this concentration step is probably critical for other viral protein-protein interactions leading to assembly, budding and maturation of the new viral particles. Nonetheless, host rafts are probably not used only as concentration devices, but also to regulate transcriptional activity of the viral genome. Further, HIV fails to assemble and bud correctly in murine cells, suggesting that these cells either lack a cellular factor needed for budding or contain a factor that inhibits this process (Mariani. R. et al (2000) J. Virol. 74, 3859-3870). It is tempting to speculate that such an inducer or inhibitor may be selectively confined or excluded from raft domains.

Chemokines are chemotactic cytokines that are released by a wide variety of cells to attract macrophages, T cells, eosinophils, basophils and neutrophils to sites of inflammation (reviewed in Schall, (1991) Cytokine, 3, 165-183 and Murphy, (1994) Rev. Immun., 12, 593-633). There are two classes of chemokines, C-X-C(alpha) and C-C (beta), depending on whether the first two cysteines are separated by a single amino acid (C-X-C) or are adjacent (C-C). The alpha-chemokines, such as interleukin-8 (IL-8), neutrophil-activating protein-2 (NAP-2) and melanoma growth stimulatory activity protein (MGSA) are chemotactic primarily for neutrophils, whereas beta-chemokines, such as RANTES, MIP-1alpha, MIP-1beta , monocyte chemotactic protein-1 (MCP-1), MCP-2, MCP-3 and eotaxin are chemotactic for macrophages, T-cells, eosinophils and basophils (Deng, et al., (1996) Nature, 381, 661-666).

Chemokines bind specific cell-surface receptors belonging to the family of G-protein-coupled seven-transmembrane-domain proteins (reviewed in Horuk, (1994) Trends Pharm. Sci., 15, 159-165) termed "chemokine receptors". On binding their cognate ligands, chemokine receptors transduce intracellular signals though associated trimeric G proteins, resulting in a rapid increase in intracellular calcium concentration. There are at least sixteen human chemokine receptors that bind or respond to beta-chemokines with the following characteristic pattern: CCR-1 ("CKR-1", "CC-CKR-1") [MIP-1alpha, MIP-1beta, MCP-3, RANTES] (Ben-Barruch et al., (1995) J. Biol. Chem., 270, 22123-22128; Beote, et al, (1993) Cell, 72, 415-425); CCR-2A and CCR-2B (or "CKR-2A"/"CKR-2A" or "CC-CKR-2A"/"CC-CKR-2A") [MCP-1, MCP-3, MCP-4]; CCR-3 (or "CKR-3" or "CC-CKR-3") [eotaxin, RANTES, MCP-3] (Combadiere, et al., (1995) J. Biol. Chem., 270, 16491-16494; CCR-4 (or "CKR-4" or "CC-CKR-4") [(MIP-lalpha, RANTES, MCP-1] (Power, et al., (1995) J. Biol. Chem., 270, 19495-19500); CCR-5 (or "CKR-5" or "CC-CKR-5") [MIP-1alpha, RANTES, MIP-1beta] (Sanson, et al., (1996) Biochemistry, 35, 3362-3367); CCR-6 (LARC); CCR-7 (ELC, SLC); CCR-8 (I-309, LEC); CCR-9 (TECK); CCR-10 (ILC, MEK); CCR-11 (ELC, SLC, TECK); CXCR-1 (ENA-78, GCP-2, IL-8); CXCR-2 (GROalpha, GRObeta, GROgamma, ENA-78,NAP-2, IL-8); CXCR-3 (Mig, IP-10, I-TAC); CXCR-4 (SDF-1); CXCR-5 (BLC), CXCR-6 (CXCL-16) and the Duffy blood-group antigen [RANTES, MCP-1] (Chaudhun, et al., (1994) J. Biol. Chem., 269, 7835-7838). beta-chemokines include eotaxin, MIP ("macrophage inflammatory protein"), MCP ("monocyte chemoattractant protein") and RANTES ("regulation-upon-activation, normal T expressed and secreted").

Human immunodeficiency virus (HIV-1), a retrovirus, is the etiological agent of a complex disease that includes progressive destruction of the immune system (acquired immune deficiency syndrome; AIDS) and degeneration of the central and peripheral nervous system. This virus was previously known as LAV, HTLV-III, or ARV.

Despite the use of available prophylactic measures, HIV-1 infection constitutes a growing pandemic, particularly in less-developed countries, for which adequate treatment is lacking. The most common therapeutic regime, highly active antiretroviral therapy (HAART), has improved the life quality of many HIV-1-infected individuals. It is nonetheless cumbersome, with serious side effects, and can also result in the emergence of drug-resistant viruses.

One focus of HIV-1 research is to understand the interplay between virus and host cell during the HIV replicative cycle, to block key interactions between viral and host proteins and prevent virus propagation without the inconveniences of HAART. Effort has concentrated on the HIV-1 entry and budding processes, which require the formation of large clusters between viral and host cell proteins (Mañes, S. et al (2003) Nat. Rev. Immunol. 3:557-568). Results suggest that HIV-1 entry into and exit from the host cell require actin cytoskeleton rearrangement and adequate cholesterol levels in host and viral membranes (Mañes, S. et al (2000) EMBO Rep. 1:190-196; Nguyen, D., and J. Hildreth. (2000) J. Virol. 74:3264-3272; Ono, A., and E. Freed. (2001) Proc. Natl. Acad. Sci. USA 98:13925-13930; del Real, G. et al (2002) J. Exp. Med. 196:293-301; Popik, W. et al (2002) J. Virol. 76:4709-4722; Nguyen, D., and D. Taub. (2002) J. Immunol. 168:4121-4126; Guyader, M. et al (2002) J. Virol. 76:10356-10364; Campbell, S. et al (2002) AIDS 16:2253-2261; Zheng, Y. et al (2003) Proc. Natl. Acad. Sci. USA 100:8460-8465; Iyengar, S. et al (1998) J. Virol. 72:5251-5255; Viard, M. et al (2002) J. Virol. 76:11584-11595; Steffens, C., and T. Hope. (2003) J. Virol. 77:4985-4991). A means remains to be found for specific targeting of these host factors to prevent HIV-1 propagation with minimal toxicity.

Certain compounds have been demonstrated to inhibit the replication of HIV, including soluble CD4 protein and synthetic derivatives (Smith, et al., (1987) Science, 238, 1704-1707), dextran sulfate, dyes such as Direct Yellow 50, Evans Blue, and certain azo dyes (U.S. Pat. No. 5,468,469). Some of these antiviral agents have been shown to act by blocking the binding of gp120, the coat protein of HIV, to its target, the CD4 glycoprotein of the cell.

Entry of HIV-1 into a target cell requires cell-surface CD4 and additional host cell cofactors. Fusin has been identified as a cofactor required for infection with virus adapted for growth in transformed T-cells, however, fusin does not promote entry of macrophagetropic viruses which are believed to be the key pathogenic strains of HIV in vivo. It has recently been recognized that for efficient entry into target cells, human immunodeficiency virus requires a chemokine receptor, most probably CCR-5 or CXCR4, as well as the primary receptor CD4 (Levy, N. (Nov. 14 1996) Engl. J. Med., 335(20), 1528-1530). The principal cofactor for entry mediated by the envelope glycoproteins of primary macrophage-trophic strains of HIV-1 is CCR5, a receptor for the beta-chemokines RANTES, MIP-1alpha and MIP-1beta (Deng, et al., (1996) Nature, 381, 661-666). HIV attaches to the CD4 molecule on cells through a region of its envelope protein, gp120. It is believed that the CD-4 binding site on the gp120 of HIV interacts with the CD4 molecule on the cell surface, and undergoes conformational changes that allow it to bind to another cell-surface receptor, such as CCR5 and/or CXCR-4. This brings the viral envelope closer to the cell surface and allows interaction between gp41 on the viral envelope and a fusion domain on the cell surface, fusion with the cell membrane, and entry of the viral core into the cell. It has been shown that beta-chemokine ligands prevent HIV-1 from fusing with the cell (Dragic, et al., (1996) Nature, 381, 667-673). It has further been demonstrated that a complex of gp120 and soluble CD4 interacts specifically with CCR-5 and inhibits the binding of the natural CCR-5 ligands MIP-1alpha and MIP-1beta (Wu et al., (1996) Nature, 384, 179-183; Trkola, et al., (1996) Nature, 384, 184-187).

Humans who are homozygous for mutant CCR-5 receptors that do not serve as co-receptors for HIV-1 in vitro, appear to be unusually resistant to HIV-1 infection and are not immuno-compromised by the presence of this genetic variant (Nature, (1996) 382, 722-725). Absence of CCR-5 appears to confer substantial protection from HIV-1 infection (Nature, (1996) 382, 668-669). Other chemokine receptors may be used by some strains of HIV-1 or may be favored by non-sexual routes of transmission. Although most HIV-1 isolates studied to date utilize CCR-5 or fusin, some can use both as well as the related CCR-2B and CCR-3 as co-receptors (Nature Medicine, (1996) 2(11), 1240-1243). Nevertheless, drugs targeting chemokine receptors may not be unduly compromised by the genetic diversity of HIV-1 (Zhang, et al., (1996) Nature, 383, 768). Accordingly, an agent which could block chemokine receptors in humans who possess normal chemokine receptors should prevent infection in healthy individuals and slow or halt viral progression in infected patients. By focusing on the host's cellular immune response to HIV infection, better therapies towards all subtypes of HIV may be provided. These results indicate that inhibition of chemokine receptors presents a viable method for the prevention or treatment of infection by HIV and the prevention or treatment of AIDS.

Eotaxin, RANTES, MIP-1alpha, MIP-1beta, MCP-1, and MCP-3 are known to bind to chemokine receptors. As noted above, the inhibitors of HIV-1 replication present in supernatants of CD8+ T cells have been characterized as the beta-chemokines RANTES, MIP-1alpha and MIP-1beta.

Most of the evidence summarized above indicates that HIV entry requires the active participation of CD4, co-receptors, and other possible participants such as glycosphingolipids, which in turn may induce the clustering of lipid rafts in an actin cytoskeleton dependent manner. Rho GTPases (a family of twenty proteins in mammals) are molecular switches that control a wide variety of eukaryotic signal transduction pathways. They are known principally for their pivotal role in regulating the actin cytoskeleton, but their ability to influence cell polarity, microtubule dynamics, membrane transport pathways and transcription factor activity may be equally significant. Rho GTPases control complex cellular processes by cycling between a GTP-bound "active" conformational state and a GDP-bound "inactive" state. Hydrolysis of GTP to GDP regulates the interconversion. In the "on" (GTP) state, GTPases recognize target proteins and generate a response until GTP hydrolysis returns the switch to the 'off' state. Signal transduction by Rho GTPases is absolutely dependent upon C-terminal prenylation. The three major Rho GTPases (Rho, Rac, and Cdc42) are geranylgeranylated postranslationally, a reaction catalyzed by the enzyme geranyl transferase. Isoprenylation of Rho GTPases permits the membrane attachment, subcellular localization, and intracellular trafficking of these proteins.

Because isoprenylation is required for function, it has been proposed that Rho GTPases are targets for drugs that prevent or reduce the synthesis of isoprenoids.

Statins are potent inhibitors of 3-hydroxy-3-methylglutaryl coenzyme A (HMG-CoA) reductase and are used to treat hypercholesterolemia with few side-effects. HMG CoA reductase is the enzyme that transfers HMG-CoA to L-mevalonic acid, the rate limiting step in cholesterol biosynthesis. L-mevalonic acid is a precursor for cholesterol biosynthesis and for generation of isoprenoids that modify specific cell proteins post-translationally. By inhibiting L-mevalonic acid synthesis, statins also prevent the synthesis of other important isoprenoid intermediates in the cholesterol biosynthetic pathway, including farnesylpyrophosphate (FPP) and geranylgeranylpyrophosphate (GGPP). Indeed, statins induce changes in the actin cytoskeleton and assembly of focal adhesion complexes by inhibiting RhoA and Rac1 isoprenylation. Rho GTPases, which must be prenylated at their C terminus for function, are molecular switches that cycle between GTP-bound (active) and GDP-bound (inactive) states to control actin cytoskeleton remodeling in response to stimuli (Etienne-Manneville, S., and A. Hall. (2002) Nature 420:629-635). By targeting HMG-CoA, statins block cholesterol biosynthesis, but also affect actin cytoskeleton rearrangement by inhibiting Rho GTPases (Koch, G. et al (1997) J. Pharmacol. Exp. Ther. 283:901-909).

Accordingly, a need exists for means to inhibit the ability of HIV and other virus from exploiting raft domain structures in the cells of humans and other animals as means of entering the cells and propagating additional virus particles.

Other objects and advantages will become apparent from the following disclosure.

### SUMMARY OF INVENTION

The present invention provides pharmaceutical compositions that inhibit the entry of human immunodeficiency virus (HIV) into eukaryotic cells. An aspect of the present invention provides pharmacological compositions that inhibit the production of HIV virions by HIV-infected cells. An aspect of the present invention provides pharmaceutical compositions containing a compound that reduces cellular pools of isoprenoid intermediates and/or inhibits protein isoprenylation and/or inhibits Rho GTPase activity.

In particular, the present invention relates to the use of a protein isoprenylation inhibitor or of a pharmaceutically acceptable salt, solvate or derivative thereof for the manufacture of a medicament for the treatment of a HIV infection, a retroviral infection genetically related to HIV, or AIDS.

From another aspect, the present invention encompasses the use of a protein isoprenylation inhibitor or of a pharmaceutically acceptable salt or solvate thereof for the manufacture of a medicament for the treatment of a HIV infection, a retroviral infection genetically related to HIV, or AIDS.

It will be appreciated that the protein isoprenylation inhibitor may be an inhibitor of geranyl geranyl pyrophosphate synthase, geranyl geranyl transferase or an inhibitor of Rho activation. As such, preferred inhibitors are statins and analogues thereof, including but not limited to lovastatin, simvastatin, pravastatin, mevastatin, atorvastatin and fluvastatin.

Optionally, the protein isoprenylation inhibitor may be admixed with a pharmaceutically acceptable carrier, binder, filler, vehicle, diluent, or excipient or any combination thereof.

In another embodiment of the invention, the protein isoprenylation inhibitor is administered in combination with one or more other therapeutic agent selected from the group comprising an HIV protease inhibitor, a non-nucleoside reverse transcriptase inhibitor, a nucleoside/nucleotide reverse transcriptase inhibitor, a CCR5 antagonist, an integrase inhibitor, an RNaseH inhibitor, a raft domain inhibitory agent, a cholesterol reducing agent, a protein prenylation reducing agent, a Rho-A GTPase inhibitor, and a glycosphingolipid reducing agent.

Examples of suitable a glycosphingolipid reducing agent include D-t-3',4'-ethylenedioxy-1-phenyl-2-palmitoylamino-3-pyrrolidino-1-propanol, D-t-4'-hydroxy-1-phenyl-2-palmitoylamino-3-pyrrolidino-1-propanol, 1-phenyl-2-palmitoylamino-3-pyrrolidino-1-propanol, pharmaceutically acceptable salts thereof, and mixtures thereof.

In a preferred embodiment, the raft domain inhibitory agent dissociates raft domains. In another preferred embodiment, the raft domain inhibitory agent inhibits the formation of raft domains.

In an alternative embodiment, the chemokine receptor modulatory agent inhibits the formation of and/or dissociates membrane raft domains.

In a yet further embodiment, the Rho-A GTPase inhibitor may be a statin or analogue thereof, including but not limited to lovastatin, simvastatin, pravastatin, mevastatin, atorvastatin and fluvastatin.

It will be appreciated that the combination of one or more of the agents may be administered in a separate, sequential or simultaneous manner.

It will also be appreciated that the one or more agents may be admixed with a pharmaceutically acceptable carrier, binder, filler, vehicle, diluent, or excipient or any combination thereof.

From another aspect, the present invention resides in a protein isoprenylation inhibitor or a pharmaceutically acceptable salt, solvate or derivative thereof for use in the treatment of a HIV, a retroviral infection genetically related to HIV, or AIDS.

A yet further aspect of the present invention provides a monotherapy for treating a human infected with HIV by administering to such a human a composition containing a pharmaceutically-effective amount of a compound that reduces cellular pools of isoprenoid intermediates and/or inhibits protein isoprenylation and/or inhibits Rho GTPase activity.

In particular, the present invention provides a method of treatment of a mammal suffering from HIV, a retroviral infection genetically related to HIV, or AIDS which comprises treating said mammal with a therapeutically effective amount of one or more agents capable of inhibiting protein isoprenylation, or a pharmaceutically acceptable salt, solvate or derivative thereof.

The present invention is directed to a combination therapy comprising administering to a patient in need of such therapy a pharmacological composition which inhibit the entry of human immunodeficiency virus (HIV) into target cells and is of value in the prevention of infection by HIV, the treatment of infection by HIV and the prevention and/or treatment of the resulting acquired immune deficiency syndrome (AIDS). The present inventive composition further comprises compounds that reduce the levels of cellular isoprenoids or inhibit protein isoprenylation or inhibit Rho-A activity, compounds that possess antiviral activity, modulators of chemokine receptor activity, glycosphingolipid reducing agents, and cholesterol reducing agents. The present invention also relates to pharmaceutical compositions containing the compounds and to a method of use of the present composition for the prevention and treatment of AIDS and viral infection by HIV.

An aspect of the present invention provides that modulators of chemokine receptor activity are specifically agents that act to disrupt raft domain organization. Such disruption includes actions to inhibit raft formation and actions to dissociate prior-assembled rafts.

An aspect of the present invention provides non-raft mutant variants of HIV-1 receptors. Such non-raft mutants are genetically-engineered variants of HIV receptors such as CD4, CXCR4, CCR5, or other receptor implicated in the HIV infection process.

An aspect of the present invention provides genetically engineered peptides that target raft domains and inhibits fusion of HIV virus to the membrane.

An aspect of the present invention inhibits targeting of HIV to raft domains. Mutant HIV receptors are provided wherein the virus-receptor binding interaction proceeds normally, but the HIV-bound receptor does not cluster in the raft domains. Thus receptor dimerization, a prerequisite to virus entry into the cell fails to take place.

An aspect of the present invention provides non-raft mutant HIV-1 receptors, wherein such mutant receptors are CD4, CXCR4, CCR5, or any other receptor implicated in HIV cellular entry.

An aspect of the present invention provides a method of treating a patient suffering from HIV infection comprising administering to the patient a pharmaceutically acceptable composition comprising a pharmaceutically effective amount of a geranyl-geranyl pyrophosphate reducing agent or other agents that inhibit geranylgeranylization of Rho GTPases.

An aspect of the present invention provides a method of treating a patient suffering from HIV infection comprising administering to the patient a pharmaceutically acceptable composition comprising a pharmaceutically effective amount of an antiviral agent, and a geranyl-geranyl pyrophosphate reducing agent or other agents that inhibit geranylgeranylization of Rho GTPases.

An aspect of the present invention provides a method of treating a patient suffering from HIV infection comprising administering to the patient a pharmaceutically acceptable composition comprising a pharmaceutically effective amount of an antiviral agent, a pharmaceutically effective amount of a protein isoprenylation reducing agent, a pharmaceutically effective amount of a modulator of chemokine receptor activity, and a pharmaceutically effective amount of a glycosphingolipid reducing agent.

An aspect of the present invention provides a method of treating a patient suffering from HIV infection comprising administering to the patient a pharmaceutically acceptable composition comprising a pharmaceutically effective amount of a known protein isoprenylation reducing agent, a pharmaceutically effective amount of a modulator of chemokine receptor activity, and a pharmaceutically effective amount of a known glycosphingolipid reducing agent.

An aspect of the present invention provides a means of treating a patient suffering from Ebola virus infection comprising administering to such a patient a pharmacological composition which inhibits the entry of Ebola virus into target cells and is of value in the prevention of infection by Ebola virus, the treatment of infection by Ebola virus, and the prevention and/or treatment of the resulting acquired disease syndrome. The present inventive composition further comprises compounds that possess antiviral activity, that are modulators of chemokine receptor activity, glycosphingolipid reducing agents, and protein isoprenylation reducing agents. The present invention also relates to pharmaceutical compositions containing the compounds and to a method of use of the present composition for the prevention and treatment of viral infection by Ebola and the sequelae thereto.

### BRIEF DESCRIPTION OF DRAWINGS

Included in the drawing are the following figures:
Figure 1 depicts membrane rafts enriched in GPI- proteins, anchored to the external leaflet of the bilayer, and enriched in acylated proteins anchored to the internal leaflet;
Figure 2 shows membrane rafts to be devices that control protein-protein interactions at the cell surface;
Figure 3 shows protein interactions enabling HIV-1 entry into rafts;
Figure 4 shows the assembly of new HIV -1 particles occurs in rafts;

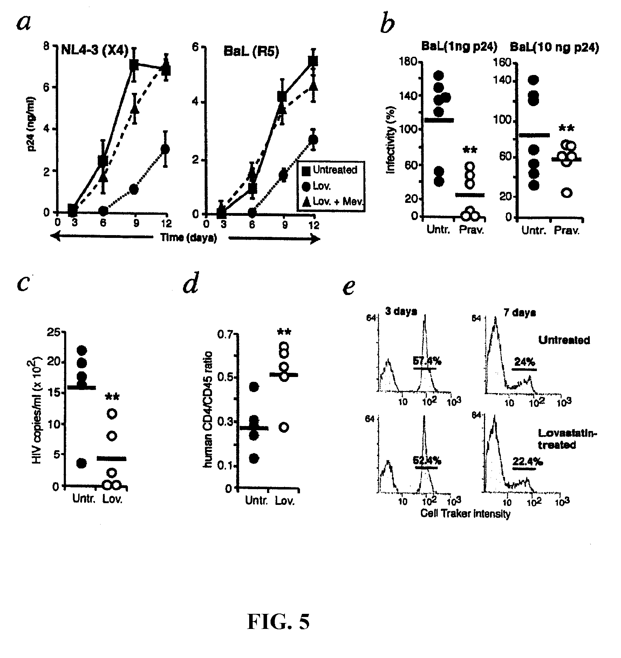

Figure 5 illustrates that statins inhibit *in vitro* and *in vivo* HIV-1 infection of human PBMC. **A**, Infection of untreated (■), lovastatin- (•) or lovastatin + mevalonate-treated (▲) PHA-activated human PBMC by X4 or R5 HIV-1 viral strains. Data are mean ± SD of triplicate points (n = 3). **B**, PBMC isolated from vehicle- or pravastatin-treated human volunteers were exposed to two doses of BaL HIV-1 strain. Data are the ratio between post- and pre-treatment p24 levels for PBMC from each individual, expressed as a percentage (**p<0.05). **C-D,** SCID mice reconstituted with human PBMC were treated with lovastatin for two weeks prior to HIV-1 infection; viral load (C) and human CD4/CD45 ratio (D) was determined for each animal one week post-infection. One representative experiment of two is shown (**p<0.05). E, Lovastatin-treated SCID mice were reconstituted with CellTracker-stained PBMC and peritoneal cell labeling examined at indicated times; numbers show the percentage of labeled cells;

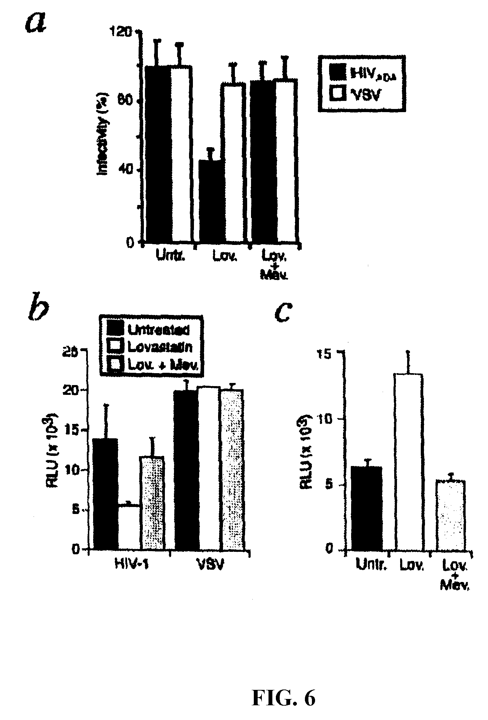

Figure 6 shows that Statins inhibit HIV-1 entry and exit. A, Single-round infections were performed in untreated, lovastatin- and lovastatin + mevalonate-treated MT2-CCR5 cells using a replication-defective NL4-3 virus pseudotyped with HIV-1_{Ada}, or VSV-G envelopes. Cell infection was normalized using untreated cells as 100%. B, Virus production was measured by titration of viral stocks produced in untreated, lovastatin- and lovastatin + mevalonate-treated HEK-293T cells transfected with a replication-defective NL4-3 virus as in A). RLU were calculated after normalization with luciferase activity from extracts of stock-producing cells. **C,** LTR-driven gene expression was analyzed in untreated, lovastatin- or lovastatin + mevalonate-treated Jurkat cells transfected with pLTR-Luc, pcDNA-tat and promoterless renilla for normalization. All data are mean ± SD of duplicate points (n = 3);

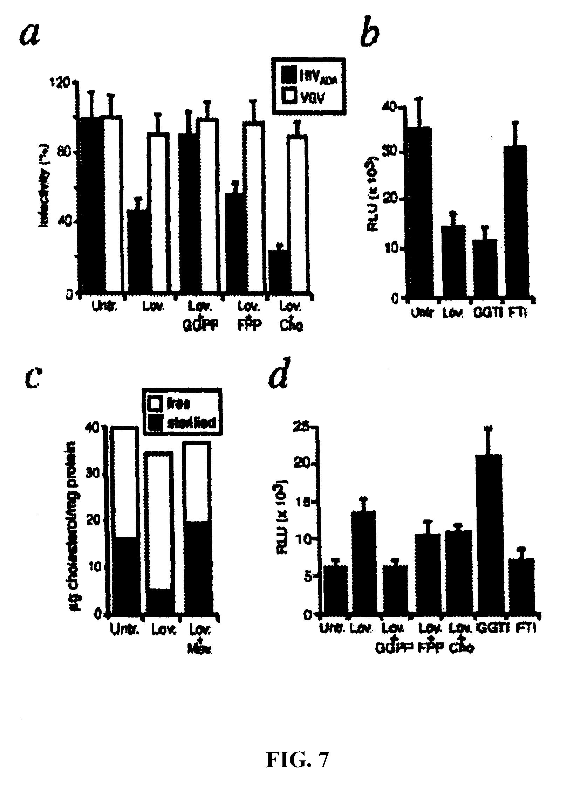

Figure 7 illustrates that the statin effects are reversed by GGPP addition. **A,** Single-round infection experiments were performed using replication-defective NL4-3 virus as in Fig. 2A, in MT2-CCR5 cells treated with lovastatin or lovastatin plus the indicated compounds. Cell infection was normalized considering untreated cells as 100%. Data are mean ± SD of duplicate points (n = 4). **B,** Single-round infections performed with the HIV-1_{Ada}-pseudotyped virus in MT2-CCR5 cells treated with lovastatin, GGTI-286 (a geranyl transferase inhibitor) or FTI-277 (a farnesyl transferase inhibitor). RLU are mean ± SD of duplicate points (n = 3). **C,** Free or esterified cholesterol levels in untreated, lovastatin-, or lovastatin + mevalonate-treated MT2-CCR5 cells. A representative experiment of two is shown. **D,** LTR-driven gene expression in MT2-CCR5 cells treated with lovastatin, lovastatin plus the indicated compounds, or with GGTI-286 or FTI-277. Data are mean ± SD of duplicates (n = 3);

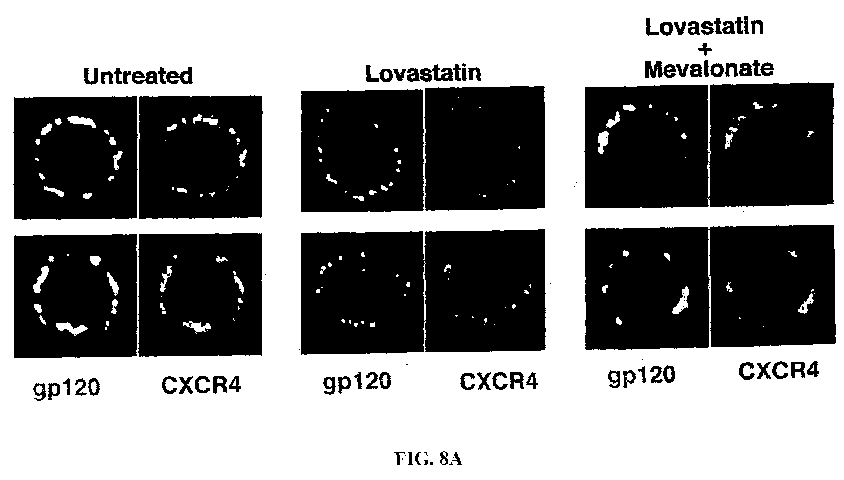

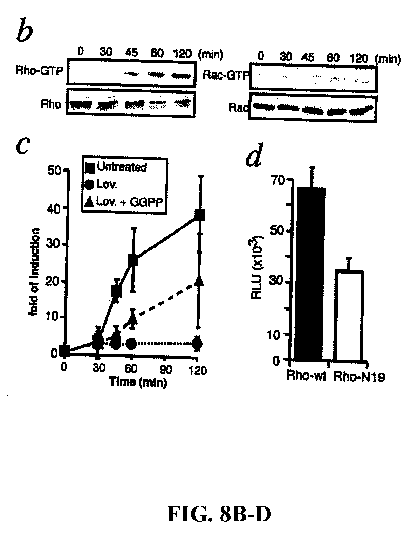

Figure 8 shows that statins inhibit gp120-induced lateral association of CD4 and CXCR4 by preventing Rho activation. **A**, Untreated, lovastatin- or lovastatin + mevalonate-treated PBMC were incubated with recombinant gp120_{IIIB} and patched with anti-gp120. After fixing, cells were stained with anti-CXCR4 and analyzed by confocal microscopy. Two representative cells are shown for each condition (n = 25). Bar, 2 µm. **B,** Serum-starved MT2-CCR5 cells were incubated with HIV-1 and cell lysates assayed for active Rho or Rac. Total Rho or Rac was analyzed in parallel in crude cell extracts as a protein loading control. One experiment of three is shown. **C,** Active Rho was determined in untreated (■), lovastatin- (•) or lovastatin + GGPP-treated cells (▲), as above. Western blots from three independent experiments were quantified by densitometry and values normalized using Rho in crude cell extracts as a loading control. Data points are plotted relative to mean values of cells not exposed to virus (time 0) for each condition. **D,** Single-round infections of MT2-CCR5 cells transfected with wild-type Rho or mutant Rho-N19 using a HIV-1_{Ada}-pseudotyped, replication-defective virus. **E,** HeLa-CD4 cells transfected with wild-type Rac, wild-type Rho, Rac-N17 or Rho-N19 were mixed with HIV gp160-expressing BSC40 cells, and cell fusion events measured. D, E, data are mean ± SD of duplicate points (n = 3).

### DETAILED DESCRIPTION

The present invention relates to the use of a protein isoprenylation inhibitor or of a pharmaceutically acceptable salt, solvate or derivative thereof for the manufacture of a medicament for the treatment of a HIV infection, a retroviral infection genetically related to HIV, or AIDS. The protein isoprenylation inhibitor may be an inhibitor of geranyl geranyl pyrophosphate synthase, geranyl geranyl transferase or an inhibitor of Rho activation. Such an inhibitor may be a statin or an analogue thereof, such as lovastatin, simvastatin, pravastatin, mevastatin, atorvastatin and fluvastatin

The present invention also relates to a therapy comprising administering to a patient in need of such therapy a pharmacological composition which inhibits the entry of human immunodeficiency virus (HIV) into target cells and is of value in the prevention of infection by HIV, the treatment of infection by HIV and the prevention and/or treatment of the resulting acquired immune deficiency syndrome (AIDS). The present inventive composition further comprises compounds that inhibit protein isoprenylation by lowering the cellular pool of isoprenoids, and/or that prevent activation of Rho-GTPases.

The present invention relates to a combination therapy comprising administering to a patient in need of such therapy a pharmacological composition which inhibit the entry of human immunodeficiency virus (HIV) into target cells and is of value in the prevention of infection by HIV, the treatment of infection by HIV and the prevention and/or treatment of the resulting acquired immune deficiency syndrome (AIDS). The present inventive composition further comprises compounds that possess antiviral activity (such as HIV protease inhibitors, non-nucleoside reverse transcriptase inhibitors, nucleoside/nucleotide reverse transcriptase inhibitors, CCR5 antagonists, integrase inhibitors and RNaseH inhibitors), that are modulators of chemokine receptor activity, glycosphingolipid reducing agents, and protein isoprenylation reducing agents. The present invention also relates to pharmaceutical compositions containing the compounds and to a method of use of the present composition for the prevention and treatment of AIDS and viral infection by HIV. Combination therapies for the prevention or treatment of HIV infection are known in the art. For example U.S. Patent 6,432,981 describes a combination therapy comprising an antiviral agent, a cholesterol lowering agent, and a modulator of chemokine receptor activity.

The present invention provides an advance over the art by targeting the cell membrane and specifically raft domains therein to prevent entry and/or budding of HIV virions. The present invention also provides an advance in targeting HIV entry and budding by regulating the clustering of raft domains. An embodiment of the present invention provides an effective dose of a pharmaceutically acceptable compound that inhibits the synthesis of sphingoglycolipids. An embodiment of the present invention provides an effective dose of an inhibitor of chemokine receptor activity. An embodiment of the present invention provides an effective dose of a cholesterol-lowering compound. An embodiment of the present invention provides an effective dose of a protein isoprenylation inhibitor. A further embodiment of the present invention provides an effective dose of an antiviral compound.

Chemokine receptor inhibitory agent. The utility of the compounds in accordance with the present invention as inhibitors of chemokine receptor activity may be demonstrated by methodology known in the art, such as the assay for chemokine binding as disclosed by Van Riper, et al., J. Exp. Med., 177, 851-856 (1993) which may be readily adapted for measurement of CCR-5 binding, and the assay for CCR-3 binding as disclosed by Daugherty, et al., J. Exp. Med., 183, 2349-2354 (1996). Cell lines for expressing the receptor of interest include those naturally expressing the receptor, such as EOL-3 or THP-1, or a cell engineered to express a recombinant receptor, such as Jurkat T lymphoblast cell line transfected with CCR5.

The utility of the compounds in accordance with the present invention as inhibitors of the spread of HIV infection in cells may be demonstrated by methodology known in the art, such as the HIV quantitation assay disclosed by Nunberg, et al., (1991) J. Virology, 65 (9), 4887-4892.

In particular, chemokine modulators, and more particularly modulators of CCR5 are disclosed in U.S. Patent 6,432,981. Further modulators of chemokine receptor activity are disclosed in U.S. Patent 6,441,001. 3-hydroxy-3-methyl-glutaryl-coenzyme A reductase inhibitors / cholesterol and isoprenoid reducing agents. Statin drugs (see Keri et al. U.S. 6,444,452) are currently the most therapeutically effective drugs available for reducing the level of LDL in the blood stream of a patient at risk for cardiovascular disease. This class of drugs includes, *inter alia*, compactin, lovastatin, simvastatin, pravastatin, mevastatin, atorvastatin and fluvastatin. The mechanism of action of statin drugs has been elucidated in some detail. They disrupt the synthesis of cholesterol and other sterols in the liver by competitively inhibiting 3-hydroxy-3-methyl-glutaryl-coenzyme A reductase (HMG-CoA reductase). HMG-CoA reductase catalyzes the conversion of HMG-CoA to L-mevalonic acid, which is the rate-determining step in the biosynthesis of cholesterol. Consequently, its inhibition leads to a reduction in the rate of formation of cholesterol in the liver. Recent experimental and clinical data nonetheless indicate that the overall benefits of statin therapy may exceed its cholesterol-lowering properties. These additional effects rely on the ability of statins to inhibit the synthesis of isoprenoid intermediates in the cholesterol biosynthetic pathway. The cell utilizes these isoprenoid intermediates for postranslational modification of various cellular proteins, including the small GTPases of the Rho and Ras superfamily. Consequently, statin-mediated HMG-CoA reductase inhibition leads to a reduction both in the rate of formation of cholesterol in the liver, as well as in protein isoprenylation. Because membrane anchoring and activation of Rho GTPases is dependent on modification by isoprenoids, statins may also prevent Rho GTPase function.

Pravastatin is the common medicinal name of the chemical compound [1S-[lalpha(beta*, delta*)2alpha, 6alpha, 8beta(R*),8aalpha]]-1,2,6,7,8,8a-hexahydro-beta, delta,6-trihydroxy-2-methyl-8-(2-methyl-1-oxobutoxy)-1-naphthalene-heptanoic acid. (CAS Registry No. 81093-370.) Pravastatin exhibits an important therapeutic advantage over other statins. A pharmaceutically effective amount of Pravastatin selectively inhibits cholesterol synthesis in the liver and small intestine but leaves cholesterol synthesis in the peripheral cells substantially unaffected. (Koga, T. et al. (1990) Biochim. Biophys. Acta, , 1045, 115-120). This selectivity appears to be due, in part, to the presence of a hydroxyl group at the C-6 position of the hexahydronaphthalene nucleus. The C-6 position is occupied by a hydrogen atom in compactin and a methyl group in lovastatin. Pravastatin is less able to permeate the lipophilic membranes of peripheral cells than the other more lipophilic congeners. (Serajuddin et al., (1991) J Pharm. Sci., 80, 830-34). The limited mobility of pravastatin is thought to account for its more localized action in the liver and intestine.

The pharmaceutical composition of the present invention preferably includes at least one statin as a cholesterol lowering agent and/or protein isoprenylation and/or Rho GTPase inhibitor. However, the cholesterol or isoprenoid or Rho GTPase lowering/inhibitory agent of the inventive pharmaceutical composition is not limited to a statin.

Antiviral agent. An antiviral agent is defined to be any substance that inhibits the biological activity of viral DNA polymerase, viral genome transcription, RNA polymerase, reverse transcriptase, helicase, primase, integrase; or inhibits viral protein translation, maturation, the formation (developing) of viral regulatory protein, or viral structural protein and the like. The viral protease inhibitors are also included herein. Preferably, an antiviral agent inhibits the biological activity, or activities, of retroviruses. Most preferably, the antiviral agent inhibits the biological activity, or activities of HIV. Examples of suitable antiviral agents include HIV protease inhibitors, non-nucleoside reverse transcriptase inhibitors, nucleoside/nucleotide reverse transcriptase inhibitors, CCR5 antagonists, integrase inhibitors and RNaseH inhibitors. On the basis of chemical structure, known antiviral agents are chiefly purine and pyrimidine derivatives, nucleosides and nucleotides. The present invention is not limited to known antiviral agents. The present invention envisions the use of further antiviral agents as they become known. Without limitation, suitable antiviral nucleosides and nucleotides include:

acyclovir: 9-[(2-hydroxyethoxy)methyl]-9H-guanine,

valacyclovir: L-valyl ester of acyclovir,

pencyclovir: 9-[4-hydroxy-3-(hydroxymethyl)-but-1-yl]guanine,

famcyclovir: diacetyl ester of pencyclovir,

gancyclovir: 9-(1,3-dihydroxy-2-propoxymethyl)guanine,

idoxuridine: 2'-deoxy-5-iodouridine,

floxuridine: 2'-deoxy-5-fluoruridine,

sorivudine: 1beta-D-arabinofuranosyl-E-5-(2-bromovinyl)uracil,

trifluridine: 5-trifluoromethyl-2'-deoxyuridine,

vidarabine: 9beta-D-ribofuranosyladenine,

zidovudine (AZT): 3'-azido-3'-deoxythymidine,

didanosine: 2',3'-dideoxyinosine,

zalcytabine: 2',3'-dideoxycytidine,

cytarabine: 4-amino-1-D-arabinofuranosyl-2(1H)-pyrimidinone,

dideoxyadenosine: 2',3'-dideoxyadenosine, and

edoxudine: 2'-deoxy-5-ethyluridine.

An antiviral agent can be used also in the form of its therapeutically useful acid addition salt, if its chemical structure allows the preparation of an acid addition salt. Similarly; the antiviral agent may be used as its therapeutically suitable salt, e.g. metal salt, ammonium salt or salts formed with organic bases, when its chemical structure is suitable for the preparation of such salts.

Nucleoside and nucleotide derivatives suitable as the antiviral agent of the present invention are disclosed in United States Patent 6,451,851, the entire contents of which is hereby incorporated by reference. A preferred embodiment of the pharmaceutical composition of the present invention comprises a nucleoside preferably zidovudine as the antiviral agent.

Protease inhibitors: HIV replication involves the synthesis of a long polypeptide chain that contains many proteins. These protein precursers, termed Gag and Gag-Pol, must be cleaved by an HIV-specific protease at 9 specific points in order to produce functional proteins. The gag precurser will eventually give rise to structural proteins and pol precurser will give rise to enzymes such as reverse transcriptase, integrase, and protease. The HIV protease is not found in mammalian cells. The HIV protease is an aspartyl protease and is unique in that it can cleave between a phenylalanine and tyrosine or proline, a reaction not catalyzed by human enzymes. HIV-Protease-specific inhibitors block the cleavage of Gag and Pol, thereby interfering with the production of new virus particles. Any pharmacologically acceptable HIV protease inhibitor is suitable for the present invention.

Pyrimidinone and pyridinone derivatives. It is known that some pyrimidinone and pyridinone derivatives inhibit HIV reverse transcriptase. In particular, derivatives of 1-[(2-hydroxyethoxy)methyl]-6-(phenylthio)thymine (HEPT) are well known for their HIV1 reverse transcriptase inhibitory properties. European Patent Application EP-0 462 800 (Merck and Company Inc.) discloses pyridinones substituted on position 3 with an aryl or heterocyclic group, linked to the pyridinone ring through a chain. Dolle et al. disclose 4-aryl-thio-pyridinones (1995, J. Med. Chem., 38, 4679-4686, and in the corresponding PCT Patent Application WO 97/05 113). Bisagni (U.S. 6,451,822) discloses 3-(amino- or aminoalkyl) pyridinone derivatives which also inhibit the reverse transcriptase of the Human Immunodeficiency Virus (HIV). Such pyrimidinone and pyridinone derivatives are suitable, but not limiting, as the antiviral agent of the present inventive pharmaceutical composition.

Glvcosphingolipid reducing agent. A glycosphingolipid reducing agent is any compound the biological action of which results in lower amounts of cellular glycosphingolipid. Hundreds of glycosphingolipids (GSLs) are derived from glucosylceramide (GlcCer), which is enzymatically formed from ceramide and UDP-glucose. The enzyme involved in GlcCer formation is UDP-glucose:N-acylsphingosine glucosyltransferase (GlcCer synthase). The rate of GlcCer formation under physiological conditions may depend on the tissue level of UDP-glucose, which in turn depends on the level of glucose in a particular tissue (Zador, I. Z. et al., (1993) J. Clin. Invest. 91:797-803). In vitro assays based on endogenous ceramide yield lower synthetic rates than mixtures containing added ceramide, suggesting that tissue levels of ceramide are also normally rate-limiting (Brenkert, A. et al., (1972) Brain Res. 36:183-193).

It has been found that the level of GSLs controls a variety of cell functions, such as growth, differentiation, adhesion between cells or between cells and matrix proteins, binding of microorganisms and viruses to cells, including binding of HIV, and metastasis of tumor cells. In addition, the GlcCer precursor, ceramide, may cause differentiation or inhibition of cell growth (Bielawska, A. et al., (1992) FEBS Letters 307:211-214). It is likely that all the GSLs undergo catabolic hydrolysis, so any blockage in the GlcCer synthase should ultimately lead to depletion of the GSLs and profound changes in the functioning of a cell or organism. An inhibitor of GlcCer synthase, PDMP (1R-phenyl-2R-decanoylamino-3-morpholino-1-propanol), previously identified as the D-threo isomer (Inokuchi, J. et al., (1987) J. Lipid Res. 28:565-571), has been found to produce a variety of chemical and physiological changes in cells and animals (Radin, N. S. et al., (1993) NeuroProtocols, A Companion to Methods in Neurosciences, S. K. Fisher et al., Ed., (Academic Press, San Diego) 3:145-155 and Radin, N. S. et al., (1993) Advances in Lipid Research; Sphingolipids in Signaling, Part B., R. M. Bell et al., Ed. (Academic Press, San Diego) 28:183-213). Compounds with longer chain fatty acyl groups have been found to be substantially more effective (Abe, A. et al., (1992) J. Biochem. 111:191-196).

Shayman et al. (U.S. Patent 6,255,336) disclose amino ceramide-like compounds that inhibit glucosyl ceramide (GlcCer) formation by inhibiting the enzyme GlcCer synthase, thereby lowering the level of glycosphingolipids. Particularly disclosed is D-t-3',4'-ethylenedioxy-1-phenyl-2-palmitoylamino-3-pyrrolidino-1-propanol and D-t-4'-hydroxy-1-phenyl-2-palmitoylamino-3-pyrrolidino-1-propanol and pharmaceutically acceptable salts thereof. Shayman et al. (U.S. Patent 6,051,598) discloses the use of 1-phenyl-2-palmitoylamino-3-pyrrolidino-1-propanol and pharmaceutically acceptable salts thereof.

Non-Raft HIV-1 Receptor Mutants: CD4, CXCR4, and CCR5 are receptors that are implicated in HIV cellular entry. Wild type receptors are incorporated into raft domains. An embodiment of the present invention provides modified receptors, mutated such that, while retaining HIV binding activity, the receptors are not localized to raft domains and/or they do not promote raft clustering. Such mutants are generated by mutation of the natural receptors using standard procedures of genetic engineering (Molecular Cloning: A laboratory manual. (1989). J. Sambrook, E.F. Fritsch, T. Maniatis Eds. Cold Spring Harbor Laboratory Press).

As an example, CD4-LDL is an artificial HIV-1 receptor, localized in non-raft domains, to which the virus binds with the same affinity as to the natural CD4 receptor. The CD4-LDL receptor does not mediate HIV-1 entry in CD4-negative cells, and functions as a decoy receptor in CD4-positive lymphocytes (del Real et al. (2002) J. Exp. Med. 196, 193-301). The CD4-LDL receptor was generated by cloning the extracellular CD4 domain in HindIII/KpnI-digested pcDNA3.1A (Invitrogen) by PCR using 5'-GCCAAGCTTATGAACCGGGGAGTC-3' [SEQ ID NO: 1] and 5'-AGAGGTACCCATTGGCTGCACCGG-3' [SEQ ID NO: 2] to produce pCD4ext. The trans- and juxtamembrane domains of the low density lipoprotein receptor (LDL-R) were rescued from pLGFP-GT46 using 5'-GCAACGGTACCGCTCTGTCCATTG-3' [SEQ ID NO: 3] and 5'-CTACTCGAGGTTCTTAAGCCGCCA-3' [SEQ ID NO: 4], and cloned in KpnI/EcoRI-opened pCD4ext. Consequently, the CD4-LDL chimera contains the amino acid sequence corresponding to the extracellular domain of the natural CD4 receptor, followed by the synthetic sequence: GTALSIVLPIVLLVFLCLGVFLLWKNWRLKN [SEQ ID NO: 5]. The artificial protein, pLGFP-GT46, contains the signal sequence of rabbit lactase-phlorizin hydrolase, the GFP, a consensus N-glycosylation site, the transmembrane domain of the human LDL-receptor generated by PCR [5'-CTGTACAAGCTTAACGGATCCAAGCTTCAGCGGCCGCACCAAGCTCTGG GCGA-3' [SEQ ID NO: 6] (forward primer) and 5'-CTTGTACAGGTTCTTAAGCCGCCAGT TCTT-3' [SEQ ID NO: 7] (reverse primer)], and the cytoplasmic tail of CD46 (Maisner et al. (1997)). Membrane cofactor protein (CD46) is a basolateral protein that is not endocytosed.

Raft-targeted fusion inhibitors: Fusion between host and viral membranes is the result of a conformational change in viral gp41, which results in the formation of a coiled-coil helix. Formation of the six-helix bundle can be inhibited by addition of peptides based on the gp41 carboxy-terminal helical domain. These peptides bind to the amino-terminal triple-stranded coiled-coil in gp41, blocking the formation of the six-helix bundle. These peptides include: 
T-20 (YTSLIHSLIEESQNQQEKNEQELLELDKWASLWNWF) [SEQ. ID.
NO: 8],
 
DP-207
(MERDREINNYTSLIHSLIEESQNQQEKNEQELLELDKWASLWNWF) [SEQ. ID.
NO: 9],
 DP-208 (LIEESQNQQEKNEQELLELDKWASLWNWF) [SEQ. ID. NO: 10], 
DP-209 (HSLIEESQNQQEKNEQELLELDKWASLWNWF) [SEQ. ID. NO:
11],
 
DP-210 (LIHSLIEESQNQQEKNEQELLELDKWASLWNWF) [SEQ. ID. NO:
12],
 
DP-211 (TSLIHSLIEESQNQQEKNEQELLELDKWASLWNWF) [SEQ. ID.
NO: 13], and
 DP-213 (LIHSLIEESQNQQEKNEQELLELDKWASL) [SEQ. ID. NO: 14] (Wild et al. (1994) Proc Natl Acad Sci USA 91: 9770-9774; Kilby et al. (1998) Nat. Med. 4: 1302-1307).

An embodiment of the present invention provides modified gp41-based peptides such that they are concentrated in raft domains, by introducing a GPI-consensus sequence at their C-terminus. The Applicant has previously showed that GPI signals effectively anchor peptides to the external leaflet of raft domains (del Real et al. (2002) J. Exp. Med. 196: 293-301). As shown in Figure 8A, HIV-1 fusion is prevented by the peptide: YTSLIHSLIEESQNQQEKNEQELLELDKWASLWNWFYDPRPSSGHSRYALIPIPL AVITTCIVLYMNVL [SEQ. ID. NO: 15], resulting from the fusion of T20 and the GPI-attachment sequence from lymphocyte function-associated antigen 3 (LFA-3; Seed, B. 1987. An LFA-3 cDNA encodes a phospholipid-linked membrane protein homologous to its receptor CD2. Nature 329: 840-842). The LFA-3-derived GPI consensus signal is a preferred amino acid sequence of the present invention. However, the GPI signal of the inventive chimeras is not limited to LFA-3.

Raft-targeted protease inhibitors: The active HIV-1 protease has a homodimeric structure, in which the subunits are connected by a beta-sheet interface formed by the N- and C-terminal amino acid segments. Short peptides derived from these segments are able to inhibit the protease activity. These peptides include ISYEL [SEQ. ID. NO: 16], YEL [SEQ. ID. NO: 17], FSYEL [SEQ. ID. NO: 18], TVSYEL [SEQ. ID. NO: 19], and QVSQNY [SEQ. ID. NO: 20] (Schramm et al. (1999) Biol Chem 380: 593-596; Schramm et al. (1996) Antiviral Res 30: 155-170).

An embodiment of the present invention provides modified HIV protease- derived peptides such that they are concentrated in raft domains, by introducing a double palmitoylation consensus sequence at their N-terminus. This signal has been shown to target cytosolic proteins to the internal leaflet of raft domains (Lacalle et al. (2002) J Cell Biol 157: 277-289). As shown in Fig. 8B, production of Luc-Ada viral particles is severely diminished in cells expressing the peptide MGCGCSSHPEDDISYEL [SEQ. ID. NO: 21], corresponding to the fusion of the 12 amino acids from the Lck unique domain and the ISYEL [SEQ. ID. NO: 16] HIV-1 protease peptide using conventional genetic engineering techniques. Likewise, Gag processing is severely attenuated in cells expressing this chimeric peptide. The Lck- derived palmitoylation consensus signal is a preferred amino acid sequence of the present invention. However, the double acylation signal of the inventive chimeras is not limited to Lck.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "pharmaceutically acceptable salts" includes pharmaceutically acceptable salts, solvates or derivatives (wherein derivatives include complexes, polymorphs, prodrugs and isotopically-labeled compounds, as well as salts, solvates and salt solvates thereof), and isomers thereof, of the disclosed compounds.

In a further embodiment, the compounds of the invention include statins and pharmaceutically acceptable salts and solvates thereof. It is to be understood that the aforementioned compounds of the invention include polymorphs and isomers thereof.

Pharmaceutically acceptable salts of the compounds of the invention include the acid addition and base salts thereof.

Suitable acid addition salts are formed from acids which form non-toxic salts. Examples include the acetate, aspartate, benzoate, besylate, bicarbonate, bisulphate, borate, bromide, camsylate, carbonate, chloride, citrate, edisylate, esylate, formate, fumarate, gluceptate, gluconate, glucuronate, hexafluorophosphate, hibenzate, hydrobromide, hydrochloride, hydroiodide, iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, sulphate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, Weinheim, Germany, 2002).

The pharmaceutically acceptable salts of the present invention may be prepared from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, the disclosure of which is hereby incorporated by reference.

The pharmaceutical composition of the invention may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or in any combination thereof). Generally, they will be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the invention and methods for their preparation will be readily apparent to those skilled in the art. Such compositions and methods for their preparation may be found, for example, in 'Remington's Pharmaceutical Sciences', 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 wt% to 5 wt% of the tablet, and glidants may comprise from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 wt% to 10 wt%, preferably from 0.5 wt% to 3 wt% of the tablet.

Other possible ingredients include anti-oxidants, colourants, flavours, preservatives and taste-masking agents.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Verma et al, (2001) Pharmaceutical Technology On-line, 25(2), 1-14. The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the compound. Examples of such formulations include drug-coated stents and PGLA microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, (October 1999) 88 (10), 955-958 by Finnin and Morgan.

Other means of topical administration include delivery by electroporation, iontophoresis, phonophoresis, sonophoresis and microneedle or needle-free (e.g. Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound comprising, for example, ethanol (optionally, aqueous ethanol) or a suitable alternative agent for dispersing, solubilising, or extending release of the compound, the propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or HPMC), blisters and cartridges for use in an inhaler or insufflator may be formulated to contain a powder mix of the compound of the invention, a suitable powder base such as lactose or starch and a performance modifier such as *l*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 microgram to 20 milligrams of the compound of the invention per actuation and the actuation volume may vary from 1 microlitre to 100 microlitres. A typical formulation may comprise a compound of the invention, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly(DL)-lactic-coglycolic acid (PGLA). Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 1 microgram to 10 milligrams of the compound of the invention. The overall daily dose will typically be in the range 1 microgram to 200 milligrams which may be administered in a single dose or, more usually, as divided doses throughout the day.

The compounds encompassed by the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds included in the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The protein isolprenylation inhibitors of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a protein isoprenylation inhibitor in combination with another therapeutic agent, for example, for the purpose of treating a particular disease or condition, it is within the scope of the present invention that two or more pharmaceutical compositions, at least one of which contains a protein isoprenylation inhibitor, may conveniently be combined in the form of a kit suitable for coadministration of the compositions.

Thus the kit of the invention comprises two or more separate pharmaceutical compositions, at least one of which contains protein isoprenylation inhibitor or a pharmaceutically acceptable salt, solvate or derivative thereof, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, having a weight of about 65 to 70 kg, the total daily dose of protein isoprenylation inhibitor is typically in the range 1 to 10000 mg, such as 10 to 1000 mg, for example 25 to 500 mg, depending, of course, on the mode of administration, the age, condition and weight of the patient, and will in any case be at the ultimate discretion of the physician. The total daily dose may be administered in single or divided doses.

Accordingly in another aspect the invention provides a pharmaceutical composition including protein isoprenylation inhibitor or a pharmaceutically acceptable salt, solvate or derivative thereof together with one or more pharmaceutically acceptable excipients, diluents or carriers.

Protein isoprenylation inhibitors, pharmaceutically acceptable salts, solvates and derivatives thereof may be administered alone or as part of a combination therapy. Thus included within the scope of the present invention are embodiments comprising coadministration of, and compositions which contain, in addition to a compound of the invention, one or more additional therapeutic agents. Such multiple drug regimens, often referred to as combination therapy, may be used in the treatment and prevention of infection by human immunodeficiency virus, HIV. The use of such combination therapy is especially pertinent with respect to the treatment and prevention of infection and multiplication of the human immunodeficiency virus, HIV, and related pathogenic retroviruses within a patient in need of treatment or one at risk of becoming such a patient. The ability of such retroviral pathogens to evolve within a relatively short period of time into strains resistant to any monotherapy which has been administered to said patient is well known in the literature. A recommended treatment for HIV is a combination drug treatment called Highly Active Anti-Retroviral Therapy, or HAART. HAART combines three or more HIV drugs. Thus, the methods of treatment and pharmaceutical compositions of the present invention may employ a protein isoprenylation inhibitor in the form of monotherapy, but said methods and compositions may also be used in the form of combination therapy in which one or more protein isoprenylation inhibitors are coadministered in combination with one or more additional therapeutic agents such as those described in detail further herein and above.

In a further embodiment of the invention, combinations of the present invention include treatment with a protein isoprenylation inhibitor, or a pharmaceutically acceptable salt, solvate or derivative thereof, and one or more additional therapeutic agents selected from the following: HIV protease inhibitors, including but not limited to indinavir, ritonavir, saquinavir, nelfinavir, lopinavir, amprenavir, atazanavir, tipranavir, AG1859 and TMC 114; non-nucleoside reverse transcriptase inhibitors (NNRTIs), including but not limited to nevirapine, delavirdine, capravirine, efavirenz, GW-8248, GW-5634 and TMC125; nucleoside/nucleotide reverse transcriptase inhibitors, including but not limited to zidovudine, didanosine, zalcitabine, stavudine, lamivudine, abacavir, adefovir dipivoxil, tenofovir and emtricitabine; CCR5 antagonists, including but not limited to: N-{(1S)-3-[3-(3-isopropyl-5-methyl-4H-1,2,4-triazol-4-yl)-*exo*-8-azabicyclo[3.2.1]oct-8-yl]-1-phenylpropyl}-4,4-difluorocyclohexanecarboxamide or a pharmaceutically acceptable salt, solvate or derivative thereof; methyl 1-*endo*-{8-[(3*S*)-3-(acetylamino)-3-(3-fluorophenyl)propyl]-8-azabicyclo[3.2.1]oct-3-yl}-2-methyl-1,4,6,7-tetrahydro-5*H-*imidazo[4,5-*c*]pyridine-5-carboxylate or a pharmaceutically acceptable salt, solvate or derivative thereof; ethyl 1-*endo*-{8-[(3S)-3-(acetylamino)-3-(3-fluorophenyl)propyl]-8-azabicyclo[3.2.1]oct-3-yl}-2-methyl-4,5,6,7-tetrahydro-1*H*-imidazo[4,5-c]pyridine-5-carboxylate or a pharmaceutically acceptable salt, solvate or derivative thereof; Sch-D, ONO-4128, GW-873140, AMD-887 and CMPD-167; integrase inhibitors, including but not limited to L-870,810; entry (e.g. fusion) inhibitors, including but not limited to enfuviritide; other agents which inhibit the interaction of gp120 with CD4, including but not limited to BMS806 and BMS-488043; and RNaseH inhibitors.

There is also included within the scope the present invention, combinations of a protein isoprenylation inhibitor, or a pharmaceutically acceptable salt, solvate or derivative thereof, together with one or more additional therapeutic agents independently selected from the group consisting of proliferation inhibitors, e.g. hydroxyurea; immunomodulators, such as granulocyte macrophage colony stimulating growth factors (e.g. sargramostim), and various forms of interferon or interferon derivatives; other chemokine receptor agonists/antagonists, such as CXCR4 antagonists (e.g. AMD-070 and AMD-3100); tachykinin receptor modulators (e.g. NK1 antagonists) and various forms of interferon or interferon derivatives; inhibitors of viral transcription and RNA replication; agents which influence, in particular down regulate, CCR5 receptor expression; chemokines that induce CCR5 receptor internalisation such MIP-1α, MIP-1β, RANTES and derivatives thereof; and other agents that inhibit viral infection or improve the condition or outcome of HIV-infected individuals through different mechanisms.

Agents which influence (in particular down regulate) CCR5 receptor expression include immunosupressants, such as calcineurin inhibitors (e.g. tacrolimus and cyclosporin A); steroids; agents which interfere with cytokine production or signalling, such as Janus Kinase (JAK) inhibitors (e.g. JAK-3 inhibitors, including 3-{(3R,4R)-4-methyl-3-[methyl-(7H-pyrrolo[2,3-d]pyrimidin-4-yl)-amino]-piperidin-1-yl}-3-oxo-propionitrile) and pharmaceutically acceptable salts, solvates or derivatives thereof; cytokine antibodies (e.g. antibodies that inhibit the interleukin-2 (IL-2) receptor, including basiliximab and daclizumab); and agents which interfere with cell activation or cell cycling, such as rapamycin.

There is also included within the scope the present invention, combinations of a protein isoprenylation inhibitor, or a pharmaceutically acceptable salt, solvate or derivative thereof, together with one or more additional therapeutic agents which slow down the rate of metabolism of the compound of the invention, thereby leading to increased exposure in patients. Increasing the exposure in such a manner is known as boosting. This has the benefit of increasing the efficacy of the compound of the invention or reducing the dose required to achieve the same efficacy as an unboosted dose. The metabolism of the compounds of the invention includes oxidative processes carried out by P450 (CYP450) enzymes, particularly CYP 3A4 and conjugation by UDP glucuronosyl transferase and sulphating enzymes. Thus, among the agents that may be used to increase the exposure of a patient to a compound of the present invention are those that can act as inhibitors of at least one isoform of the cytochrome P450 (CYP450) enzymes. The isoforms of CYP450 that may be beneficially inhibited include, but are not limited to, CYP1A2, CYP2D6, CYP2C9, CYP2C19 and CYP3A4. Suitable agents that may be used to inhibit CYP 3A4 include, but are not limited to, ritonavir, saquinavir or ketoconazole.

It will be appreciated by a person skilled in the art, that a combination drug treatment, as described herein above, may comprise two or more compounds having the same, or different, mechanism of action. Thus, by way of illustration only, a combination may comprise a compound of the invention and: one or more NNRTIs; one or more NRTIs and a PI; one or more NRTIs and a CCR5 antagonist; a PI; a PI and an NNRTI; and so on.

In addition to the requirement of therapeutic efficacy, which may necessitate the use of therapeutic agents in addition to the protein isoprenylation inhibitors, there may be additional rationales which compel or highly recommend the use of a combination of a compound of the invention and another therapeutic agent, such as in the treatment of diseases or conditions which directly result from or indirectly accompany the basic or underlying disease or condition. For example, it may be necessary or at least desirable to treat Hepatitis C Virus (HCV), Hepatitis B Virus (HBV), Human Papillomavirus (HPV), opportunistic infections (including bacterial and fungal infections), neoplasms, and other conditions which occur as the result of the immune-compromised state of the patient being treated. Other therapeutic agents may be used with the compounds of the invention, e.g., in order to provide immune stimulation or to treat pain and inflammation which accompany the initial and fundamental HIV infection.

Accordingly, therapeutic agents for use in combination with the compounds of formula (I) and their pharmaceutically acceptable salts, solvates and derivatives also include: interferons, pegylated interferons (e.g. peg-interferon alpha-2a and peg-interferon alpha-2b), lamivudine, ribavirin, and emtricitabine for the treatment of hepatitis; antifungals such as fluconazole, itraconazole, and voriconazole; antibacterials such as azithromycin and clarithromycin; interferons, daunorubicin, doxorubicin, and paclitaxel for the treatment of AIDS related Kaposi's sarcoma; and cidofovir, fomivirsen, foscarnet, ganciclovir and valcyte for the treatment of cytomegalovirus (CMV) retinitis.

Further combinations for use according to the invention include combination of a protein isoprenylation inhibitor, or a pharmaceutically acceptable salt, solvate or derivative thereof with a CCR1 antagonist, such as BX-471; a beta adrenoceptor agonist, such as salmeterol; a corticosteroid agonist, such fluticasone propionate; a LTD4 antagonist, such as montelukast; a muscarinic antagonist, such as tiotropium bromide; a PDE4 inhibitor, such as cilomilast or roflumilast; a COX-2 inhibitor, such as celecoxib, valdecoxib or rofecoxib; an alpha-2-delta ligand, such as gabapentin or pregabalin; a beta-interferon, such as REBIF; a TNF receptor modulator, such as a TNF-alpha inhibitor (e.g. adalimumab), a HMG CoA reductase inhibitor, such as a statin (e.g. atorvastatin); or an immunosuppressant, such as cyclosporin or a macrolide such as tacrolimus.

In the above-described combinations, the protein isoprenylation inhibitor or a pharmaceutically acceptable salt, solvate or derivative thereof and other therapeutic agent(s) may be administered, in terms of dosage forms, either separately or in conjunction with each other; and in terms of their time of administration, either simultaneously or sequentially. Thus, the administration of one component agent may be prior to, concurrent with, or subsequent to the administration of the other component agent(s).

Accordingly, in a further aspect the invention provides a pharmaceutical composition comprising a protein isoprenylation inhibitor or a pharmaceutically acceptable salt, solvate or derivative thereof and one or more additional therapeutic agents.

It is to be appreciated that all references herein to treatment include curative, palliative and prophylactic treatment.

The pharmaceutical composition of the invention can be also suitable for genetic therapy of HIV target cells.

In addition to primates, such as humans, a variety of other mammals can be treated according to the method of the present invention. For instance, mammals including, but not limited to, cows, sheep, goats, horses, dogs, cats, guinea pigs, rats or other bovine, ovine, equine, canine, feline, rodent or murine species can be treated. However, the method can also be practiced in other species, such as avian species (e.g., chickens).

### EXAMPLES

As illustrated below, statins inhibit HIV-1 infection of SCID mice grafted with adult human peripheral blood mononuclear cells (PBMC; SCID-hu-PBMC), an *in vivo* model of acute HIV-1 infection. The results also illustrate that statins inhibit virus entry into and exit from target cells by targeting Rho geranylation. Strikingly, one-month oral statin administration reduced serum HIV-1 RNA copy number in chronically HIV-1-infected individuals not receiving HAART (Table I). These data provide evidence to support the principle of the use of statins as therapeutic anti-retroviral agents.

**Table I. Clinical parameters of HIV-1-infected, statin-treated patients**

| Patient ID | | #1 | #2 | #3 | #4 | #5 | #6 |
|---|---|---|---|---|---|---|---|
| Sex | | Male | Male | Female | Male | Male | Male |
| Age | | 53 | 23 | 33 | 24 | 42 | 39 |
| Virus transmission | | Sexual | Sexual | IVDU¹ | Sexual | IVDU | IVDU |
| Diagnosis date | | 1997 | 2000 | 1996 | ND | 1998 | 1996 |
| HAART | | No | No | No | No | No | No |
| HCV² co-infection | | No | No | Yes | No | Yes | Yes |
| Other | | Ethylism, | | Methadone | | Methadone | Methadone |
| | | | Asthma | | No | | |
| | | pancreatitis | | treatment | | treatment | treatment |
| Viral load³ | before | | | | | 37,300 | 46,400 |
| | | 16,800 | 19,500 | 50,100 | 84,000 | | |
| | after | | | | | 21,600 | 26,300 |
| | | 2,330 | 9,940 | 12,138 | 3,590 | | |
| | rebound | | | | | 26,400 | 32,600 |
| | ⁴ | 16,100 | 56,100 | 64,000 | 26,400 | | |
| CD4⁺ (count/ml) | before | 798 | 520 | 513 | 760 | 465 | 538 |
| | after | 940 | 560 | 540 | 1,010 | 487 | 552 |
| | rebound | 690 | 550 | 501 | 501 | 478 | 560 |

| | | | | | | | |
|---|---|---|---|---|---|---|---|
| ¹ Intravenous drug use. ² Hepatitis C virus. ³ Viral load is expressed as HIV-1 RNA copies/ml, | | | | | | | |
| ⁴ Measurements after three months without treatment. | | | | | | | |

### MATERIALS AND METHODS

**HIV-1 infection.** Single-round infections were performed with a replication-defective pNL4-3.Luc.R-E pseudotyped with HIV-1_{ADA} or vesicular stomatitis virus (VSV) envelopes (Manes, S. et al (2000) EMBO Rep. 1:190-196). MT2-CCR5 cells (a gift of J. Alcamí, Inst. Salud Carlos III, Madrid, Spain) were treated with 10 µM lovastatin (48 h, 37°C) alone or combined with L-mevalonate (200 µM), geranylgeranylpyrophosphate (GGPP; 5 µM), farnesylpyrophosphate (FPP; 5 µM) or cholesterol (5 µg/ml, all from Sigma), or with GGTI-286 (10 µM) or FTI-277 (10 µM, both from Calbiochem) before transduction with viral supernatants (0.1 multiplicity of infection; 2 h, 37°C). Infectivity was determined after 24 h by measuring luciferase activity (Mañes, S. et al (2000) EMBO Rep. 1:190-196; del Real, G. et al (2002) J. Exp. Med 196:293-301). Similar experiments were performed using MT2-CCR5 cells expressing GFP-tagged wild type Rho, wild type Rac, or the Rho-N19 or Rac-N17 mutants (a gift of F. Sánchez-Madrid, Hospital de la Princesa, Madrid, Spain). gp160-induced cell-cell fusion assays were as described (Mañes, S. et al (2000) EMBO Rep. 1:190-196).

PBMC purified on Ficoll-Hypaque gradients (Amersham Biosciences) were activated for 2 days with phytohemagglutinin (PHA; 1 µg/ml) and IL-2 (50 ng/ml), and treated (48 h, 37°C) with lovastatin or lovastatin + mevalonate. Treated PBMC were incubated with NL4-3 or BaL viral stocks (1 or 10 ng p24 antigen/10⁶ cells; 3 h, 37°C). Cell-free supernatants were collected daily from cultured cells (0.5 x 10⁶/ml) and tested for p24 antigen (Coulter).

For *ex vivo* infection, PBMC were purified from informed pravastatin-treated donors (40 mg/day, 14 days, oral) before and after treatment. Both samples were infected simultaneously with two infectious doses of HIV-1 BaL stocks. After washing, cells (0.5 x 10⁶ cells/ml) were plated with PHA and IL-2, and p24 measured at 4 and 5 days post-infection (Coulter).

**Murine SCID-hu-PBMC model.** Eight- to 10-week-old non-leaky phenotype CB.17 SCID/SCID mice were reconstituted by i.p. injection of 50 x 10⁶ human PBMC. One week later, mice with comparable serum human immunoglobulin levels, proof of reconstitution with human cells, received lovastatin (i.p; 5 mg/kg) every three days, beginning one week before HIV-1 NL4-3 challenge (i.p.; 100 TCID₅₀/ml) until sacrifice. Plasma HIV-1 RNA copy number was measured (Amplicor HIV-1 Monitor Assay; Roche Molecular Systems) one week post-infection. Two weeks after viral challenge, peritoneal cells (10⁶) from sacrificed mice were incubated with 2 x 10⁶ PHA-activated human PBMC in the presence of IL-2, and p24 was determined after 2 weeks co-culture. Peritoneal cells were also analyzed by FACS (EPICS Elite; Coulter) using FITC-labeled anti-CD45 and phycoerythrin-anti-CD4 antibodies (Ab) (Pharmingen). Untreated or lovastatin-treated mice were reconstituted with CellTracker Green CMFDA (Molecular Probes)-stained PBMC. At 3 and 7 days after reconstitution, peritoneal cells were obtained from two mice, pooled, and analyzed by FACS.

**Titration of viral production.** HEK 293T cells, co-transfected with pNL4-3.Luc.R.E. and cDNA encoding HIV-1_{ADA} or VSV envelopes, were treated with lovastatin or lovastatin + mevalonate. Viral stocks were harvested after 48 h and titrated by measuring luciferase activity after transduction of CD4-expressing HEK 293T cells. Values were normalized to luciferase activity from extracts of stock-producing cells.

**LTR-driven gene expression.** Jurkat cells transfected with pLTR-luc (Schwartz, O. et al (1990) Gene 88:197-205), pcDNA-tat and the promoterless renilla luciferase plasmid were treated at 4 h post-transfection with inhibitors and metabolites at the indicated concentrations (see HIV-1 infections section). Relative luciferase units (RLU) were calculated as the ratio between firefly and renilla activity after 48 h.

**Cell cholesterol mass determination.** Cholesterol content of untreated, lovastatin- or lovastatin- + mevalonate-treated MT2-CCR5 cells was analyzed on a Hewlett-Packard gas chromatograph (Chrompack, Middelburg, The Netherlands) as described (Llaverias, G. et al (2002) Eur. J. Pharmacol. 451:11-17). The cholesteryl ester mass was calculated by subtracting free cholesterol from total cholesterol content.

**gp120-induced patching.** Unstimulated PBMC plated into ICAM-2/Fc (R&D Systems)-coated chambers were incubated (30 min, 12°C) with recombinant gp120 (T cell line-adapted X4 virus, isolate IIIB; Intracel) in PBS/0.2% bovine serum albumin, followed by rabbit anti-gp120 and Cy2-anti-rabbit Ab (Jackson ImmunoResearch). Cells were fixed with 3.7% paraformaldehyde in PBS on ice, then incubated sequentially with biotinylated anti-CXCR4 (FAB172; R&D Systems) and streptavidin-Cy3. Finally, cells were mounted in Vectashield medium (Vector Laboratories) and visualized by confocal laser microscopy (Leica).

**Rho and Rac activation assay.** MT2-CCR5 cells (3 x 10⁶) treated with lovastatin alone or in combination with GGPP were starved (3 h), then incubated with HIV-1 stocks. At times indicated, cells were washed with ice-cold PBS and lysates prepared using Rho or Rac activation assay kits (Upstate Biotechnology). GTP-bound Rho was precipitated with RBD-agarose beads and GTP-Rac with PBD-agarose beads. Activated Rho or Rac were measured in pellets by Western blot with specific antibodies, using crude cell extracts for normalization. Densitometry was performed using NIH Image software.

**Lovastatin treatment of HIV-1-infected patients.** Six informed HIV-1-infected patients in A1 disease stage who did not receive HAART were treated with lovastatin (40 mg/day, oral) for one month. Plasma HIV RNA copy number, circulating CD4⁺ T lymphocyte counts, and plasma cholesterol levels were measured before and immediately after treatment, as well as three months after termination of lovastatin treatment, using standard clinical techniques.

### RESULTS AND DISCUSSION

### Statins inhibit HIV-1 infection in vitro and in vivo

It is suggested that statins may have anti-HIV-1 effects (Maziere, J. et al (1994) Biomed. Pharmacother. 48:63-67). PHA-activated human PBMC, pretreated for 48 h with 10 µM lovastatin, were exposed to X4 (NL4-3) or R5 (BaL) HIV-1 strains; no cytotoxic effects were observed at this dosage (not shown). Lovastatin inhibited HIV-1 replication, as indicated by reduced p24 antigen production in X4- and R5-infected cultures (Fig. 5A). This effect was reversed by co-incubation of cells with L-mevalonate, the product of HMG CoA reductase.

To analyze the statin-induced anti-HIV-1 effect, susceptibility to R5 virus infection of PBMC from pravastatin-treated human volunteers was compared, before and after statin treatment. Infectivity of PBMC from vehicle-treated individuals was not significantly affected at two different HIV-1 doses (Fig. 5B; p = 0.812 for 1 ng of p24/10⁶ cells; p = 0.218 for 10 ng of p24/10⁶ cells, two-tailed Wilcoxon Signed-Rank test). Infectivity was drastically reduced in PBMC from statin-treated volunteers (p = 0.032 for both virus doses, two-tailed Wilcoxon Signed-Rank test).

Blockade of HIV-1 replication by statins was tested in SCID mice grafted with human PBMC (SCID-hu-PBMC), an *in vivo* HIV-1 infection model (del Real, G. et al (1998) AIDS 12:865-872), by injecting lovastatin before HIV-1 NL4-3 challenge. Mean viral load was significantly reduced in lovastatin-treated mice (p = 0.028, two-tailed Mann-Whitney test) compared to vehicle-treated animals (Fig. 5C). Viral RNA was undetectable in plasma of 4 of 10 lovastatin-treated mice; co-culture of peritoneal cells from two of these mice with PHA-activated human PBMC did not rescue virus. At one week post-infection, lovastatin-treated SCID-hu-PBMC mice showed higher CD4⁺ T cell counts than controls; the average CD4⁺/CD45⁺ ratio was 51% in lovastatin- and 28% in vehicle-treated mice (Fig. 5D), indicating specific CD4⁺ cell loss in controls (p = 0.048, two-tailed Mann-Whitney test). To determine whether statins affected viability or proliferation of specific grafted human cell populations, SCID mice were reconstituted with fluorescent-labeled, PHA-activated human cells and lovastatin-treated as above. No difference was found in the number of labeled cells or in labeling intensity (Fig. 5E), suggesting that lovastatin treatment is not deleterious for grafted PBMC.

### Statins affects the HIV-1 replicative cycle by reducing geranylgeranylation

Single-round infection with a replication-defective HIV-1 NL4-3 variant showed that lovastatin inhibited entry of R5- (Fig. 6A) or X4-pseudotyped (not shown) variants, but not that of viruses pseudotyped with the VSV envelope (Fig. 6A). Lovastatin treatment also reduced HIV-1-X4-pseudotyped viral production, but not that of VSV-G-pseudotyped viruses, by HEK 293T cells transfected with replication-defective NL4-3.Luc DNA (Fig. 6B). It is unlikely that the specific lovastatin-induced reduction in HIV-1-pseudotyped viral production is due to differential Gag synthesis and processing, since HIV-1 and VSV pseudotypes share the same viral genome. Lovastatin nonetheless increased HIV-1 LTR-driven promoter activity (Fig. 6C), suggesting that the drug can regulate the activity of nuclear factors involved in HIV transcription. These results indicate that lovastatin has pleiotropic effects on HIV-1 replication, as the drug can promote virus replication by increasing transcription of the viral genome, and it has anti-HIV-1 effects that inhibit virus entry into and exit from the target cell. Both pro- and anti-HIV-1 lovastatin-induced effects were mediated through the mevalonate pathway, as they were reversed by co-incubation of cells with L-mevalonate (Fig. 6A-C).

Inhibition of the mevalonate pathway diminishes cholesterol biosynthesis, but also reduces cell pools of GGPP and FPP, both involved in post-translational protein modification. Applicant has found that lovastatin-induced inhibition of HIV-1 entry into permissive cells was reversed by co-addition of GGPP, but not of FPP or cholesterol (Fig. 7A). This suggests that lovastatin inhibits HIV-1 infection by blocking protein geranylgeranylation rather than by preventing farnesylation or reducing cholesterol biosynthesis. R5-pseudotyped virus entry was inhibited by cell treatment with a geranylgeranyl transferase inhibitor, but not a farnesyl transferase inhibitor (Fig. 7B); these drugs did not affect VSV-pseudotyped virus entry (not shown). Measurement of cell cholesterol content indicated comparable free cholesterol levels in lovastatin-treated and untreated cells; the drug nonetheless drastically reduced the cholesteryl ester mass (Fig. 7C), probably due to concomitant inhibition of acyl-CoA:cholesterol acyltransferase (Kam, N. et al (1990) Biochem. J. 272:427-433). Whereas a role for esterified cholesterol in HIV-1 infection cannot be excluded, the fmding that GGPP reverses lovastatin-induced inhibition of virus entry suggests that lovastatin effects are mediated mainly by impairment of protein geranylgeranylation. Supplementation with GGPP, but not FPP or free cholesterol, also reversed the lovastatin-induced increase in LTR-driven transcription (Fig. 7D). Cell treatment with a geranylgeranyl transferase inhibitor also increased HIV transcription (Fig. 7D), suggesting a general molecular mechanism for lovastatin mediation of pro- and anti-HIV-1 effects.

### Statins inhibit HIV-1-induced receptor clustering by preventing Rho activation

To identify the isoprenylated protein(s) involved in statin-induced anti-HIV-1 effects, the mechanism by which lovastatin inhibits virus entry was studied. The formation of higher order molecular complexes of gp120 with the HIV-1 receptors CD4 and CXCR4 was analyzed. Untreated or lovastatin-treated PBMC were incubated sequentially with gp120_{IIIB}, anti-gp120 and anti-CXCR4 Ab. Lovastatin-treated cells had smaller gp120 patches than untreated cells (Fig. 8A); the patches co-localized with CD4 in both cases (not shown). Although lovastatin did not affect gp120 binding to CD4, co-localization between gp120 and CXCR4 was drastically reduced in lovastatin-treated cultures (Fig. 8A), suggesting that lovastatin inhibits affects gp120-induced receptor clustering. Patch size and gp120-CXCR4 co-localization were restored in lovastatin-treated cells by addition of mevalonate (Fig. 8A), but not cholesterol (not shown).

Geranylgeranylation is needed for post-translational lipid modification of several proteins anchored to the inner membrane leaflet, including the Rho GTPases (Koch, G. et al (1997) J. Pharmacol. Exp. Ther. 283:901-909). Moreover, gp120 binding to target cells modifies Rho molecular mass and increases Cdc42 expression (Cicala, C. et al (2002) Proc. Natl. Acad. Sci. USA 99:9380-9385). Target cell incubation with HIV-1 resulted in activation of Rho, but not Rac (Fig. 8B) or Cdc42 (not shown). Cell incubation with lovastatin prior to virus exposure inhibited HIV-1-induced Rho activation, which was reversed when cells were co-incubated with GGPP (Fig. 8C), indicating that lovastatin prevented HIV-1-induced Rho activation by a geranylgeranylation-dependent mechanism. Virus-induced Rho activation is required for virus entry, since infection by R5-pseudotyped HIV-1 was reduced in dominant- negative RhoN19 mutant-expressing cells (Fig. 8D); RhoN19 expression also specifically prevented HIV-1 envelope fusion with target cell membrane in a cell-cell fusion assay (Fig. 8E). The results suggest that lovastatin inhibits HIV-1 entry into target cells, at least in part, by preventing Rho activation. Rho inhibition has been associated with an increase in HIV-1 transcription (Wang, L. et al (2000) J. Immunol. 164:5369-5374), suggesting that lovastatin-induced pro- and anti-HIV-1 effects may be Rho-mediated.

### Statins reduce plasma HIV RNA copy number in chronically infected individuals

Statins are used for treatment of HAART-associated lipodystrophy. Based on the above *in vitro* results, the potential use of statins for in vivo treatment of HIV patients was tested. In a preliminary study for proof of concept, six A1 stage HIV-1-infected, non-HAART-treated patients with stable viral load for at least six months (Table I) were lovastatin-treated for one month as sole therapy. Short-term statin treatment induced a clear reduction in serum viral RNA loads in all patients (Table I). Discontinuation of statin treatment caused a rebound in viral load (Table I). The data suggest that statins can inhibit HIV-1 replication in chronically infected individuals, and support the use of statins as anti-retroviral agents.

Statins may have several immune cell targets (Romano, M. et al (2000) Lab. Invest. 80:1095-1100; Kwak, B. et al (2000) Nat. Med. 6:1399-1402). The Applicant has shown that statin-induced inhibition of HIV-1 entry and virion production, as well as the increase in viral transcription, is mediated via mevalonate pathway inhibition. HIV-1 entry and budding are cooperative processes that require protein co-aggregation at the host cell surface: CD4 and the chemokine coreceptors for entry, Gag and gp160 for budding (Mañes, S. et al (2003) Nat. Rev. Immunol. 3:557-568). It is suggested that these processes are mediated by protein association with lipid rafts (Mañes, S. et al (2000) EMBO Rep. 1:190-196; Nguyen, D., and J. Hildreth (2000) J. Virol. 74:3264-3272; Ono, A., and E. Freed (2001) Proc. Natl. Acad. Sci. USA 98:13925-13930; del Real, G. et al (2002) J. Exp. Med. 196:293-301; Wang, J.-K. et al (2000) Proc. Natl. Acad. Sci. USA 97:394-399; Lindwasser, O., and M. Resh. (2001) J. Virol. 75:7913-7924; Manes, S. et al (2001) Semin. Immunol. 13:147-157) and driven by the actin cytoskeleton (Iyengar, S. et al (1998) J. Virol. 72:5251-5255; Viard, M. et al (2002) J. Virol. 76:11584-11595; Steffens, C., and T. Hope (2003) J. Virol. 77:4985-4991). Raft clustering entails actin cytoskeleton reorganization, for which some reports implicate Rho as a key effector (Mañes, S. et al (2003) Trends Immunol. 24:320-326). Statins can inhibit HIV-1 infection in part by reducing Rho geranylgeranylation, essential for Rho localization and function, including the cytoskeletal reorganization required for virus entry and exit.

In summary, evidence has been provided that statins prevent HIV-1 infection in cultured primary cells, in animal models, and in chronically-infected individuals. It has been shown that, at the cellular level, statins inhibit viral entry and budding by preventing Rho geranylgeranylation, necessary for HIV-1 infection. Based on the ability of statins to lower viral load in HIV-1-infected individuals, we suggest that these compounds have direct anti-retroviral effects and might be appropriate drugs for more accessible treatment of the AIDS pandemic.

### INCORPORATION BY REFERENCE

All publications and patent applications cited in this specification are herein incorporated by reference as if each individual publication or patent application were specifically and individually indicated to be incorporated by reference. More specifically, the following publications are hereby incorporated in their entirety and for all purposes: G. del Real et al., *Blocking of HIV-1 Infection By Targeting CD4 to Nonraft Membrane Domains,* 196(2) J. Exp. Med. 293 (2002); S. Mañes et al., *Membrane Raft Microdomains in Chemokine Receptor Function,* 13 Seminars in Immunology 147 (2001); S. Mañes et al., *Membrane Raft Microdomains Mediate Lateral Assemblies required for HIV-1 Infection,* 1(2) EMBO Reports 190 (2000); S. Manes et al., *Pathogens: raft hijackers*, 3 Nat. Rev. Immunol. 557 (2003); and R.A. Lacalle, E. Mira, C. Gómez-Moutón, S. Jiménez-Baranda, C. Martínez-A. & S. Mañes, *Specific SHP-2 partitioning in raft domains triggers integrin-mediated signaling via Rho activation,* J. Cell Biol.157, 277-290, 2002.

The entire contents and disclosure of co-pending application, Attorney Docket Number 21910/0011, entitled Method To Screen For Chemokine Agonists And Antagonists, is specifically incorporated by reference and for all purposes.

### STATEMENT OF INDUSTRIAL UTILITY

For purposes of complying with the Patent Cooperation Treaty, the present invention states an industrial utility. The Present invention provides means of diagnosing and treating humans and other animals afflicted with a disease or a condition mediated by chemokine receptor signaling.

## Claims

1. The use of a protein isoprenylation inhibitor or of a pharmaceutically acceptable salt, solvate or derivative thereof for the manufacture of a medicament for the treatment of a HIV infection, a retroviral infection genetically related to HIV, or AIDS.

2. Use according to claim 1, wherein the protein isoprenylation inhibitor is an inhibitor of geranyl geranyl pyrophosphate synthase.

3. Use according to claim 1, wherein the protein isoprenylation inhibitor is an inhibitor of geranyl geranyl transferase.

4. Use according to claim 1, wherein the protein isoprenylation inhibitor is an inhibitor of Rho activation.

5. Use according to any one of claims 1 to 4, wherein the inhibitor is a statin or an analogue thereof.

6. Use according to claim 5, wherein the statin is selected from the group comprising lovastatin, simvastatin, pravastatin, mevastatin, atorvastatin and fluvastatin.

7. Use according to any one of claims 1 to 6, wherein in the protein isoprenylation inhibitor is admixed with a pharmaceutically acceptable carrier, binder, filler, vehicle, diluent, or excipient or any combination thereof

8. Use according to any one of claims 1 to 7, wherein the protein isoprenylation inhibitor is administered in combination with one or more other therapeutic agent selected from the group comprising an HIV protease inhibitor, a non-nucleoside reverse transcriptase inhibitor, a nucleoside/nucleotide reverse transcriptase inhibitor, a CCR5 antagonist, an integrase inhibitor, an RNaseH inhibitor, a raft domain inhibitory agent, a cholesterol reducing agent, a protein prenylation reducing agent, a Rho-A GTPase inhibitor, and a glycosphingolipid reducing agent.

9. Use according to claim 8, wherein said glycosphingolipid reducing agent is a compound selected from the group consisting of:
D-t-3',4'-ethylenedioxy-1-phenyl-2-palmitoylamino-3-pyrrolidino-1-propanol,
D-t-4'-hydroxy-1-phenyl-2-palmitoylamino-3-pyrrolidino-1-propanol,
1-phenyl-2-palmitoylamino-3-pyrrolidino-1-propanol,
pharmaceutically acceptable salts thereof, and mixtures thereof.

10. Use according to claim 8 or 9, wherein said raft domain inhibitory agent dissociates raft domains.

11. Use according to claim 8 or 9, wherein said raft domain inhibitory agent inhibits the formation of raft domains.

12. Use according to any one of claims 8 to 11, wherein said chemokine receptor modulatory agent inhibits the formation of and/or dissociates membrane raft domains.

13. Use according to any one of claims 8 to 11, wherein said Rho-A GTPase inhibitor is a statin.

14. Use according to any one of claims 8 to 13, wherein the combination comprises separate, sequential or simultaneous administration of one or more of the agents.

15. Use according to any one of claims 8 to 14, wherein in the one or more agents is admixed with a pharmaceutically acceptable carrier, binder, filler, vehicle, diluent, or excipient or any combination thereof

16. A protein isoprenylation inhibitor or a pharmaceutically acceptable salt, solvate or derivative thereof for use in the treatment of a HIV, a retroviral infection genetically related to HIV, or AIDS.

17. A method of treatment of a mammal suffering from HIV, a retroviral infection genetically related to HIV, or AIDS which comprises treating said mammal with a therapeutically effective amount of one or more agents capable of inhibiting protein isoprenylation, or a pharmaceutically acceptable salt, solvate or derivative thereof.

18. The method of claim 9 further comprising administering to the patient a pharmaceutically effective amount of at least one agent selected from the group consisting of an antiviral agent, a chemokine receptor modulatory agent, a raft domain inhibitory agent, a cholesterol reducing agent, a protein prenylation reducing agent, a Rho-A GTPase inhibitor, and a glycosphingolipid reducing agent.

19. The method of claim 17 or claim 18, wherein in the one or more agents is admixed with a pharmaceutically acceptable carrier, binder, filler, vehicle, diluent, or excipient or any combination thereof

20. The method of any one of claims 17 to 19, wherein said antiviral agent is an addition salt selected from the group consisting of an acid addition salt, a metal addition salt, an ammonium salt, and a salt formed with an organic base.

21. The method according to any one of claims 17 to 20, wherein said glycosphingolipid reducing agent is a compound selected from the group consisting of:
D-t-3',4'-ethylenedioxy-1-phenyl-2-palmitoylamino-3-pyrrolidino-1-propanol,
D-t-4'-hydroxy-1-phenyl-2-palmitoylamino-3-pyrrolidino-1-propanol,
1-phenyl-2-palmitoylamino-3-pyrrolidino-1-propanol,
pharmaceutically acceptable salts thereof, and mixtures thereof.

22. The method according to any one of claims 17 to 21, wherein said antiviral agent is a compound selected from the group consisting of nucleosides, nucleotides, protease inhibitors, pyrimidinones, and pyridinones.

23. The method according to any one of claims 17 to 22, wherein said raft domain inhibitory agent dissociates raft domains.

24. The method according to any one of claims 17 to 22, wherein said raft domain inhibitory agent inhibits the formation of raft domains.

25. The method according to any one of claims 17 to 24, wherein said chemokine receptor modulatory agent inhibits the formation of and/or dissociates membrane raft domains.

26. The method according to any one of claims 17 to 25, wherein said Rho-A GTPase inhibitor is a statin.

27. The method according to any one of claims 17 to 26, wherein the method further comprises separate, sequential or simultaneous administration of one or more of the agents.

28. A method of treatment of a mammal suffering from HIV, a retroviral infection genetically related to HIV, or AIDS by preventing the accumulation of HIV receptors in raft domains comprising providing a non-raft targeted mutant cytokine receptor.

29. The method according to Claim 28, wherein said mutant receptor binds HIV but does not enter into raft domains.
